# EUROPEAN PATENT APPLICATION

(11) **EP 4 501 908 A1**
(43) Date of publication of application: **05.02.2025**
(21) Application number: 23780775.5
(22) Date of filing: 29.03.2023
(51) Int. Cl.: C07C 319/14, C07C 323/22, C07C 323/52

(54) **POLYETHER ETHER KETONE DECOMPOSITION METHOD AND NOVEL SUBSTANCE USING STARTING MATERIAL THAT IS DECOMPOSITION PRODUCT OBTAINED BY THIS DECOMPOSITION METHOD**

(30) Priority: 29.03.2022 JP 2022053618
(71) Applicant: National Institute of Advanced Industrial Science and Technology, Chiyoda-ku Tokyo 100-8921 (JP)
(72) Inventor: MINAMI Yasunori, Tsukuba-shi, Ibaraki 305-8560 (JP); NAKAJIMA Yumiko, Tsukuba-shi, Ibaraki 305-8560 (JP); SATO Kazuhiko, Tsukuba-shi, Ibaraki 305-8560 (JP)
(74) Representative: van Dam, Vincent
(86) International application number: PCT/JP2023/012993
(87) International publication number: WO 2023/190767

(57) **Abstract**

The present invention provides a polyether ether ketone decomposition method for efficiently decomposing polyether ether ketone, and a novel compound synthesized by using a decomposition product obtained by the polyether ether ketone decomposition method as a raw material.

[Solution]

A polyether ether ketone decomposition method for decomposing polyether ether ketone, including a first reaction step of reacting the polyether ether ketone with a base and at least one of an alkanethiol, an aromatic mercaptan, sodium sulfide, and elemental sulfur in an organic solvent. Polyether ether ketone can be efficiently decomposed by this polyether ether ketone decomposition method.

## Description

### Technical Field

The present invention relates to a method for decomposing polyether ether ketone, and a novel substance made by using a decomposition product obtained by the decomposition method as a raw material.

### Background Art

Polyether ether ketone ((OC₆H₄OC₆H₄COC₆H₄)ₙ), a super engineering plastic, is a thermoplastic resin that has ideal properties including not only excellent heat resistance, high temperature properties, chemical resistance, and electrical insulation, but also high mechanical strength, dimension stability, non-flammability, and low smoke generation. Due to these property, it has a wide range of applications including: applications as a retainer ring, a wafer chucker, or a transport container in the semiconductor-related field; applications that contribute to weight reduction, improved fuel efficiency, and improved maintainability in the high-end space field; applications as an actuator, a gear, or a bearing in the automotive field; applications for energy production; applications as a compressor, a pump, or the like for chemical industry in a general industrial field; as well as applications as food processing equipment. Currently, the global production volume of polyether ether ketone is as low as approximately 100,000 tons as of 2018, but it is predicted to grow at an average annual rate of 4.54% from 2020 to 2026. Polyether ether ketone is an essential material for industrial society in the future.

However, polyether ether ketone is extremely difficult to dispose of and recycle due to its high stability. In addition, its molecular structure contains many benzene rings, making it difficult for microorganisms to decompose it.

Therefore, polyether ether ketone has a high environmental impact and poses major problems for the future. In fact, there are only a few cases targeting PPS or polyether sulfone (PESU) (Patent Literature 1; Non-Patent Literature 1, 2, 3, 4, and 5). If things continue as they are, we are unable to cope with not only the high environmental impact but also a future in which non-recyclable plastics are banned. Furthermore, even under the current circumstances, polyether ether ketone is a highpriced product, so disposing of it would result in a large economic loss.

As a related reaction, substitution of methoxy groups with amino groups by arylamines by using an organic superbase catalyst t-Bu-P₄, which is para-methoxy substituted benzophenone and one of the structural units of polyphenylene sulfide (see Non-Patent Literature 6) has been known. However, there is no report of this method being applied to the depolymerization of polyether ether ketone.

### Citation List

### Patent Literature

[PTL 1] Japanese Unexamined Patent Application Publication No. 2013-249324

### Non Patent Literature

[NPL 1] Yu, Z. L.; Miao, G. X.; Chen, Y. R. Macromol Chem Phys 1996, 197,4061.
[NPL 2] Wang, S. J.; Bian, S. G.; Yan, H.; Xiao, M.; Meng, Y. Z. J. App. Poly. Sci. 2008, 110, 4049.
[NPL 3] Lian, Z.; Bhawal, B. N.; Morandi, B. Science 2017, 356, 1059.
[NPL 4] Minami, Y; Matsuyama, N.; Matsuo, Y.; Tamura, M.; Sato, K.; Nakajima, Y. Synthesis 2021, 53, 3351.
[NPL 5] Delcaillau, T.; Woenckhaus-Alvarez, A.; Morandi, B. Org. Lett. 2021, 23, 7018.
[NPL 6] Shigeno, M.; Hayashi, K.; Nozawa-Kumada, K.; Kondo, Y. Org. Lett. 2019, 21, 5505.

### Summary of Invention

### Technical Problem

An object of the present invention is to provide a polyether ether ketone decomposition method for efficiently decomposing polyether ether ketone, as well as a novel compound and the like synthesized from a decomposition product obtained by the polyether ether ketone decomposition method.

### Solution to Problem

The present inventors have conducted intensive research into decomposing polyether ether ketone, and as a result have found a polyether ether ketone decomposition method for efficiently decomposing polyether ether ketone, and have also found that a novel compound or the like can be synthesized from a decomposition product obtained by the polyether ether ketone decomposition method.

In particular, the present inventors have found that, by reacting polyether ether ketone with 2-phenylethylthiol in the presence of sodium tert-butoxide, the polyether ether ketone can be efficiently decomposed and disodium benzophenone-4,4'-dithiolate and hydroquinone can be efficiently produced.

Moreover, the present inventors have found that an alkyl halide or an acid halide added to a decomposition product obtained by the polyether ether ketone decomposition method reacts only with disodium benzophenone-4,4'-dithiolate and convert it to a corresponding sulfur-functionalized benzophenone while leaving hydroquinone behind.

A first aspect according to the present invention is a polyether ether ketone decomposition method for decomposing polyether ether ketone, including a first reaction step of reacting the polyether ether ketone with a base and at least one of an alkanethiol, an aromatic mercaptan, sodium sulfide, and elemental sulfur in an organic solvent. Here, "elemental sulfur" refers to octasulfur (S₈).

According to the first aspect, the polyether ether ketone can be efficiently decomposed into monomers.

A second aspect according to the present invention is the polyether ether ketone decomposition method according to the first aspect, in which the at least one of the alkanethiol, the aromatic mercaptan, sodium sulfide, and elemental sulfur is reacted in an amount of 0.1 to 6 equivalents relative to polyether ether ketone.

According to the second aspect, the polyether ether ketone can be decomposed more efficiently.

A third aspect according to the present invention is the polyether ether ketone decomposition method according to the first aspect, in which the base is reacted in an amount of 2 to 6 equivalents relative to the polyether ether ketone.

According to the third aspect, the polyether ether ketone can be decomposed more efficiently.

A fourth aspect according to the present invention is the polyether ether ketone decomposition method according to any one of the first to third aspects, in which the first reaction step is carried out at 100 to 200°C.

According to the fourth aspect, the polyether ether ketone can be decomposed into monomers more efficiently without destroying functional groups in a main chain of polyether ether ketone.

A fifth aspect according to the present invention is the polyether ether ketone decomposition method according to the first aspect, in which the base is at least one selected from a group consisting of sodium hydroxide, potassium hydroxide, potassium carbonate, potassium phosphate, cesium carbonate, sodium *tert*-butoxide, lithium *tert-*butoxide, potassium *tert*-butoxide, lithium bis(trimethylsilyl)amide, sodium bis(trimethylsilyl)amide, potassium bis(trimethylsilyl)amide, a phosphazene base, *t*-Bu-P₄ (1-*tert*-butyl-4,4,4-tris(dimethylamino)-2,2-bis[tris(dimethylamino)-phosphoranylideneamino]-2λ⁵,4λ⁵-catenadi(phosphazene)), *t*-Oct-P₄ *(1-tert-octyl-4,4,4-*tris(dimethylamino)-2,2-bis[tris(dimethylamino)-phosphoranylideneamino]-2λ⁵,4λ⁵-catenadi(phosphazene)), and *t*-Bu-P₂ (1-*tert*-butyl-2,2,4,4,4-pentakis(dimethylamino)-2λ⁵,4λ⁵-catenadi(phosphazene)).

According to the fifth aspect, the polyether ether ketone can be decomposed more reliably.

A sixth aspect according to the present invention is the polyether ether ketone decomposition method according to the first aspect, in which the organic solvent is at least one selected from a group consisting of 1,3-dimethyl-2-imidazolidinone, N,N-dimethylacetamide, N,N-dimethylformamide, N-methyl-2-pyrrolidone, benzonitrile, and 1,4-dioxane.

According to the sixth aspect, the polyether ether ketone can be decomposed more reliably.

A seventh aspect according to the present invention is a polyether ether ketone decomposition method, including a second reaction step of reacting a first reaction product (first decomposition product) obtained by the first reaction step according to the first aspect with at least one of an alkyl halide, an acid halide, and hydrogen chloride.

According to the seventh aspect, useful compounds can be produced.

An eighth aspect according to the present invention is the polyether ether ketone decomposition method according to the seventh aspect, in which: the alkyl halide is at least one selected from a group consisting of methyl iodide, 1-bromohexane, benzyl bromide, 2-phenylethyl bromide, 11-bromomethyltricosane, 1-bromo-3,7-dimethyloctane, 1,4-dichlorobenzene, 4-bromo-1-butene, 3-bromobutanoic acid ethyl ester, bromomethylcyclopropane, 2-bromoethanol, and 3-bromo-1-propene oxide; and the acid halide is at least one selected from a group consisting of acetyl chloride, benzoyl chloride, and α-ethylhexanoic acid chloride.

According to the eighth aspect, a benzophenone with a desired structure can be produced from the first decomposition product obtained by the first reaction step.

A ninth aspect according to the present invention is a compound represented by any one of formulas (1) to (11), which is synthesized by using a product obtained by the polyether ether ketone decomposition method according to the first aspect.

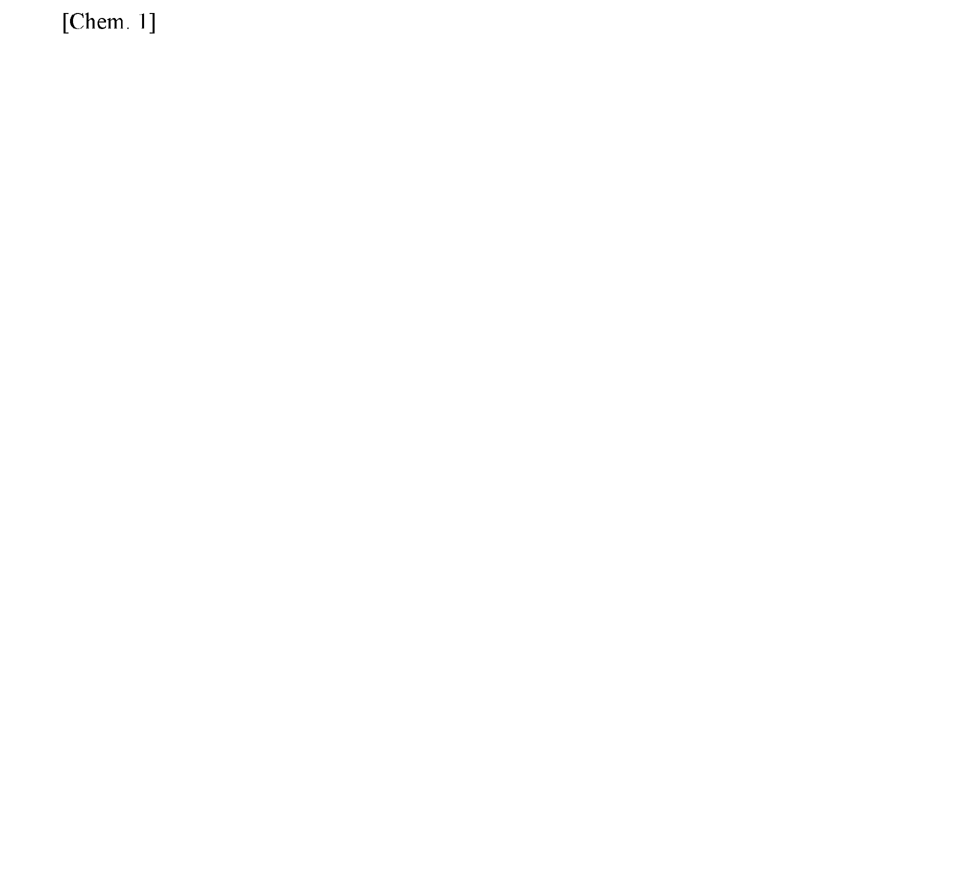

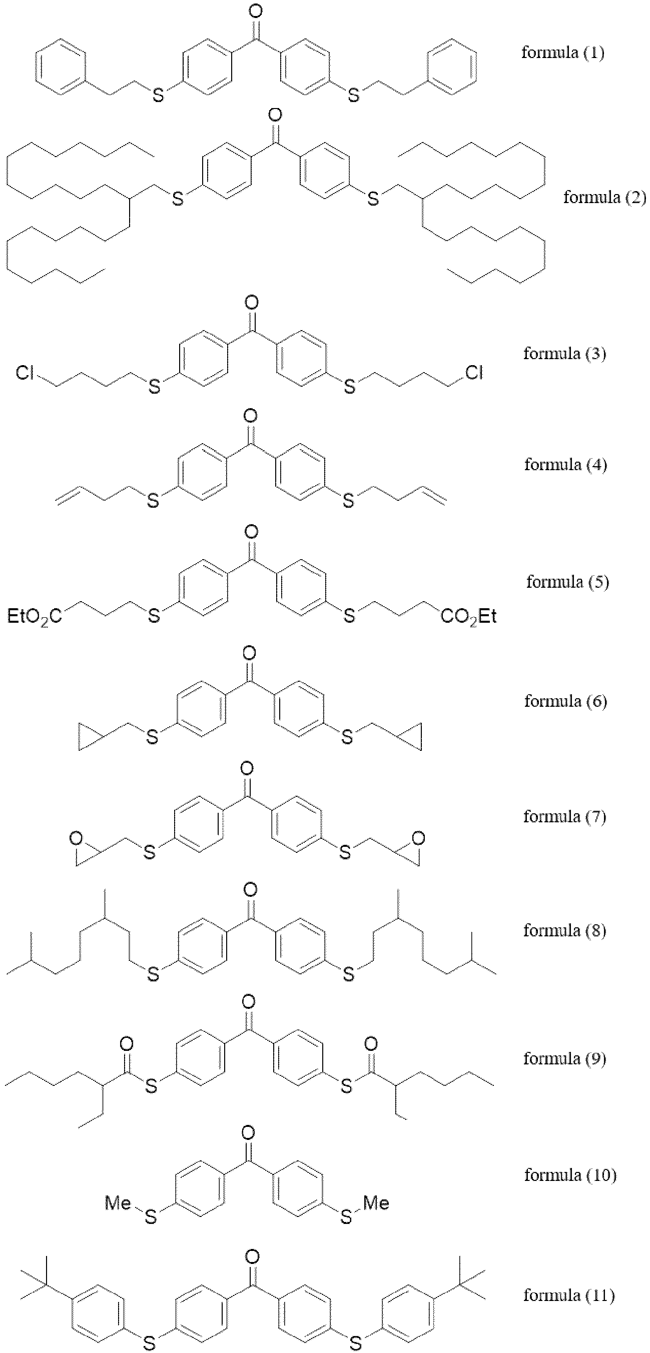

The "product" in this aspect is a concept that includes not only the first decomposition product but also other products.

According to the ninth aspect, novel compounds of the formulas (1) to (9) and (11), as well as a compound of formula (10) can be produced from the polyether ether ketone. These compounds are very useful because they serve as raw materials for functional polymers, functional plastics, and the like.

### Advantageous Effects of Invention

According to the present invention, polyether ether ketone can be efficiently decomposed. Moreover, according to the present invention, sulfur-functionalized benzophenones and/or hydroquinone including novel compounds can be produced from polyether ether ketone with high yield and high selectivity. Furthermore, according to the present invention, useful novel substances can be produced (synthesized).

### Brief Description of Drawings

[Fig. 1] Fig. 1 shows a result of ¹H NMR analysis of a first decomposition product obtained in Example 9.
[Fig. 2] Fig. 2 shows a result of ¹H NMR analysis of a first decomposition product obtained in Example 10.

### Description of Embodiments

Hereinafter, embodiments of the polyether ether ketone decomposition method according to the present invention will be described. Note that the present invention is not limited to the following embodiments.

### (Embodiment 1)

The polyether ether ketone decomposition method according to this embodiment includes:
(a) a first reaction step of reacting the polyether ether ketone (PEEK) with a base and at least one of an alkanethiol, an aromatic mercaptan, sodium sulfide, and elemental sulfur in an organic solvent; and
(b) a second reaction step of reacting a first decomposition product obtained by the first reaction step in (a) with at least one of an alkyl halide, an acid halide, and hydrogen chloride.

Polyether ether ketone is a compound with a structure in which a benzene ring is linked to a ketone at the para position via an ether bond, and can be represented by the following formula (1).

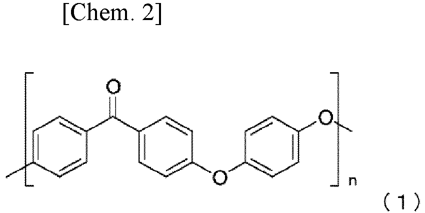

The shape of polyether ether ketone is not particularly limited and may be in the form of powder, pellet, film, or block. The molecular weight is also not particularly limited, but the weight average molecular weight is preferably from 5,000 to 50,000, more preferably from 10,000 to 30,000, and most preferably 20,800. The number average molecular weight is preferably from 1,000 to 50,000, more preferably from 5,000 to 30,000, and most preferably from 9,000 to 11,000.

As described above, the polyether ether ketone decomposition method according to this embodiment consists of two reaction steps, i.e., the first reaction step (a) and the second reaction step (b).

The first reaction step (a) can be considered to be, for example, a chemical reaction represented by the following chemical reaction formula. That is, it is a reaction in which polyether ether ketone is first decomposed into 4,4'-di(2-phenylethylthio)benzophenone, and then 4,4'-di(2-phenylethylthio)benzophenone is further decomposed into disodium benzophenone-4,4'-dithiolate.

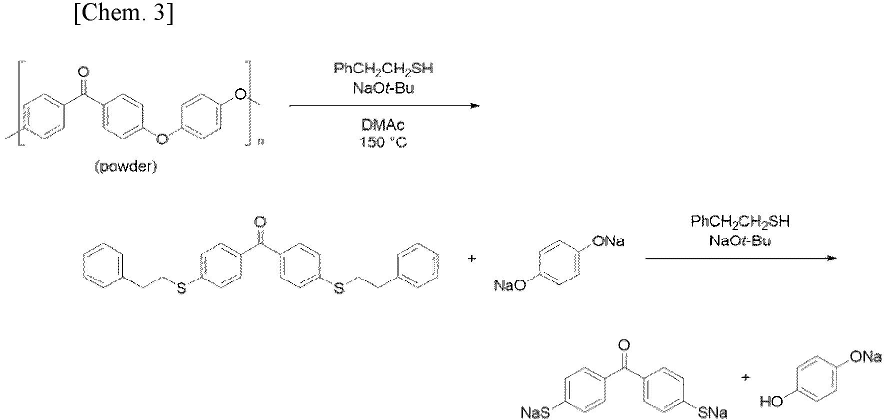

As a substance (sulfur reactant) to be reacted with polyether ether ketone in the first reaction step (a), alkanethiols, aromatic mercaptans, sodium sulfide, or elemental sulfur can be used which can react with a base such as sodium hydroxide, sodium carbonate, potassium hydroxide, potassium carbonate, potassium phosphate, cesium carbonate, lithium *tert*-butoxide, sodium *tert*-butoxide, potassium *tert*-butoxide, lithium bis(trimethylsilyl)amide, sodium bis(trimethylsilyl)amide, or potassium bis(trimethylsilyl)amide to convert a thiol to a thiolate. Note that those skilled in the art will easily understand that a thiol can be converted to a thiolate by using the following compounds and a base.

Examples of alkanethiols include ethanethiol, propanethiol, butanethiol, pentanethiol, hexanethiol, heptanethiol, octanethiol, decanethiol, dodecanethiol, tetradecanethiol, pentadecanethiol, octadecanethiol, icosanethiol, docosanethiol, 2-methylbutanethiol, 2-ethylhexanethiol, benzylthiol, p-methylbenzylthiol, o-methylbenzylthiol, α-methylbenzylthiol, phenoxyphenylmethanethiol, (4-chlorophenyl)methanethiol, (4-fluorophenyl)methanethiol, (4-methoxyphenyl)methanethiol, 4-*tert*-butylphenylmethanethiol, 2-(pyrazin-2-yl)ethane-1-thiol, (1,3-thiazol-4-yl)methanethiol, 2-phenylethylthiol, 2-(3-nitrophenyl)ethane-1-thiol, 2-(2-chlorophenyl)ethane-1-thiol, 1,2-dimercaptoethane, 1,3-propanedithiol, 1,4-decanedithiol, 1,5-pentanedithiol, 1,6-hexanedithiol, 1,8-octanedithiol, 1,10-decanedithiol, propane-1,2-dithiol, butane-1,2-dithiol, 2-mercaptoethanol, 3-mercaptopropanol, 4-mercaptobutanol, 6-mercaptohexanol, 1,4-disulfanylbutane-2,3-diol, (1-methylcyclobutyl)methanethiol, (oxan-4-yl)methanethiol, (oxolan-3-yl)methanethiol, 2-(methoxycarbonyl)ethylthiol, 2-(2,2-sulfanylethoxy)ethane-1-thiol, ethyl 2-sulfanyl acetate, 2-[(sulfanylethyl)sulfanyl]ethane-1-thiol, 2-(2,2-difluoroethoxy)ethane-1-thiol, 2-(2-cyclopropylethoxy)ethane-1-thiol, 2,2-dimethoxyethane-1-thiol, 2-[(aminoethyl)amino]ethane-1-thiol, 3-(triethoxysilyl)propane-1-thiol, 3-(trimethoxysilyl)propane-1-thiol, 2,2-dimethyl-3-sulfanylpropanol, 3-(tert-butoxy)propane-1-thiol, 3-cyclopropylpropane-1-thiol, 3-fluoropropane-1-thiol, 3-methanesulfanylpropane-1-thiol, 4,4,4-trifluorobutane-1-thiol, 2-aminoethane-1-thiol, 2-aminoethane-1-thiol hydrochloride, 2-(2-aminoethoxy)ethane-1-thiol hydrochloride, 2-aminopropane-1-thiol, 2-aminopropane-1-thiol hydrochloride, 3-dimethylaminopropanethiol, {bicyclo[3,1,0]hexan-6-yl}methanethiol, t-butylthiol, and the like. Examples of aromatic mercaptans include thiophenol, 4-methylbenzenethiol, 4-methoxybenzenethiol, 4-(t-butyl)benzenethiol, and the like, and are capable of decomposing polyether ether ketone into at least di(carbothio)benzophenone.

Among these, when an alkanethiol having a primary carbon group or benzyl group susceptible to an S_{N}2 reaction, such as ethanethiol, propanethiol, butanethiol, pentanethiol, hexanethiol, heptanethiol, octanethiol, decanethiol, dodecanethiol, tetradecanethiol, pentadecanethiol, octadecanethiol, icosanethiol, docosanethiol, 2-methylbutanethiol, 2-ethylhexanethiol, benzylthiol, p-methylbenzylthiol, o-methylbenzylthiol, α-methylbenzylthiol, phenoxyphenylmethanethiol, (4-chlorophenyl)methanethiol, (4-fluorophenyl)methanethiol, (4-methoxyphenyl)methanethiol, 4-tert-butylphenylmethanethiol, 2-(pyrazin-2-yl)ethane-1-thiol, (1,3-thiazol-4-yl)methanethiol, 2-phenylethylthiol, 2-(3-nitrophenyl)ethane-1-thiol, 2-(2-chlorophenyl)ethane-1-thiol, 1,2-dimercaptoethane, 1,3-propanedithiol, 1,4-decanedithiol, 1,5-pentanedithiol, 1,6-hexanedithiol, 1,8-octanedithiol, 1,10-decanedithiol, propane-1,2-dithiol, butane-1,2-dithiol, 2-mercaptoethanol, 3-mercaptopropanol, 4-mercaptobutanol, 6-mercaptohexanol, 1,4-disulfanylbutane-2,3-diol, (1-methylcyclobutyl)methanethiol, (oxan-4-yl)methanethiol, (oxolan-3-yl)methanethiol, 2-(methoxycarbonyl)ethylthiol, 2-(2,2-sulfanylethoxy)ethane-1-thiol, ethyl 2-sulfanyl acetate, 2-[(sulfanylethyl)sulfanyl]ethane-1-thiol, 2-(2,2-difluoroethoxy)ethane-1-thiol, 2-(2-cyclopropylethoxy)ethane-1-thiol, 2,2-dimethoxyethane-1-thiol, 2-[(aminoethyl)amino]ethane-1-thiol, 3-(triethoxysilyl)propane-1-thiol, 3-(trimethoxysilyl)propane-1-thiol, 2,2-dimethyl-3-sulfanylpropanol, 3-(tert-butoxy)propane-1-thiol, 3-cyclopropylpropane-1-thiol, 3-fluoropropane-1-thiol, 3-methanesulfanylpropane-1-thiol, 4,4,4-trifluorobutane-1-thiol, 2-aminoethane-1-thiol, 2-aminoethane-1-thiol hydrochloride, 2-(2-aminoethoxy)ethane-1-thiol hydrochloride, 2-aminopropane-1-thiol, 2-aminopropane-1-thiol hydrochloride, 3-dimethylaminopropanethiol, or {bicyclo[3,1,0]hexan-6-yl}methanethiol, or a thiol having an electron-withdrawing group at the β-position or capable of desorbing sulfur by abstraction of β-hydrogen with a base, such as 2-phenylethylthiol, is used, polyether ether ketone can be decomposed into the first decomposition product.

In the first reaction step (a), the sulfur reactant is reacted in an amount of preferably 2 to 6 equivalents, and particularly preferably 3.5 to 4.5 equivalents relative to polyether ether ketone. Note that the amount of the base is not particularly limited and is preferably 0.1 to 6 equivalents, more preferably 1 to 4 equivalents, and most preferably 2.5 to 3.5 equivalents relative to polyether ether ketone.

The organic solvent that can be used in the first reaction step (a) is not particularly limited as long as it has a boiling point higher than the temperature acting in the first reaction step described below and can dissolve the thiolate by solvation without reacting the thiolate. Examples of such organic solvents include 1,3-dimethyl-2-imidazolidinone, N,N-dimethylacetamide, N,N-dimethylformamide, N-methyl-2-pyrrolidone, benzonitrile, 1,4-dioxane, and the like.

The reaction in the first reaction step (a) can be carried out in an inert gas (e.g., argon gas, nitrogen, or the like) atmosphere, or in an air atmosphere.

The reaction in the first reaction step (a) can be carried out within a temperature range of room temperature (25°C) to 200°C, preferably 100 to 200°C, and more preferably 140 to 160°C. If the reaction temperature is lower than 25°C, the decomposition reaction does not proceed, and if the reaction temperature exceeds 200°C, benzophenones cannot be obtained as the first decomposition product.

The reaction in the first reaction step (a) is preferably carried out for 1 to 40 hours, and more preferably 20 to 24 hours. During the reaction in the first reaction step (a), the first reaction product is preferably stirred by a known means. If the reaction time in the first reaction step (a) is less than 1 hour, the reaction does not occur sufficiently, and if it exceeds 40 hours, the reaction does not proceed any further.

The first decomposition product obtained after the first reaction step (a) is preferably brought to room temperature (20 to 30°C) before being used in the subsequent second reaction step (b).

In the second reaction step (b), at least one of the alkyl halide, the acid halide, and hydrogen chloride is reacted, which can react with benzophenone-4,4'-dithiolate contained in the first decomposition product obtained from the first reaction step (a) without any additive and can form a carbon-sulfur bond.

Examples of the alkyl halide that can be used in the second reaction step (b) include methyl iodide, 1-bromohexane, benzyl bromide, 2-phenylethyl bromide, 11-bromomethyltricosane, 1-bromo-3,7-dimethyloctane, 1,4-dichlorobenzene, 4-bromo-1-butene, 3-bromobutanoic acid ethyl ester, bromomethylcyclopropane, 2-bromoethanol, 3-bromo-1-propene oxide, and the like.

Note that the alkyl halide that can be used in the second reaction step (b) of the present invention includes not only the aforementioned substances but also analogs of alkyl halides. Examples of the analogs of the alkyl halide include alkanes having oxygen functional groups (e.g., triflate groups, tosylate groups) that undergo nucleophilic reactions, such as methyl trifluoromethanesulfonate and ethyl trifluoromethanesulfonate. Note that those skilled in the art will easily understand that the analogs of the alkyl halides will react in the second reaction step (b) of the present invention in the same manner as the aforementioned alkyl halides.

Examples of the acid halide that can be used in the second reaction step (b) include acetyl chloride, benzoyl chloride, α-ethylhexanoic acid chloride, and the like. In the second reaction step (b), the alkyl halide is reacted in an amount of preferably 2 to 6 equivalents, more preferably 2 to 4 equivalents, and particularly preferably 2.7 to 3.3 equivalents relative to polyether ether ketone.

Those skilled in the art will easily understand that a similar reaction will occur when hydrogen chloride is used in place of the alkyl halide or acid halide.

The reaction in the second reaction step (b) can be carried out in air. The reaction in the second reaction step (b) can be carried out preferably at room temperature (25°C) to 200°C, and more preferably at 80 to 100°C.

The reaction in the second reaction step (b) is preferably carried out for 1 to 24 hours, and more preferably 1 to 2 hours.

During the reaction in the second reaction step (b), a reaction mixture is preferably stirred by a known means.

An organic layer of a second reaction product obtained after the reaction in the second reaction step (b) is separated by adding, for example, hydrochloric acid and ethyl acetate to the second reaction product, and the organic layer is washed with water and a saturated aqueous sodium chloride solution. Then, it is dried by using anhydrous sodium sulfate or anhydrous magnesium sulfate, and distillation under a reduced pressure can be carried out to obtain a sulfur-functionalized benzophenone and hydroquinone as crude products.

Note that, in the polyether ether ketone decomposition method according to this embodiment, a desired sulfur-functionalized benzophenone can be obtained by selecting the alkyl halide or acid halide used in the second reaction step (b) as appropriate.

An example of the sulfur-functionalized benzophenone obtained by the polyether ether ketone decomposition method according to this embodiment is a compound represented by the following formula (2).

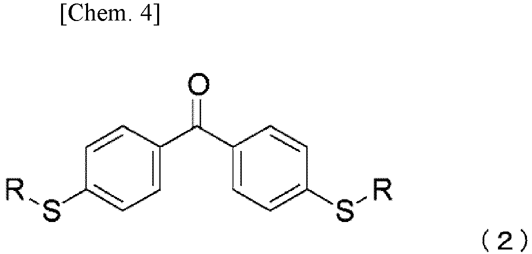

(In the formula (2), R represents a carbon group with 1 to 20 carbon atoms or a hydrogen atom. Specifically, it represents an alkyl group with 1 to 20 carbon atoms, an alicyclic alkyl group with 3 to 20 carbon atoms, an alkenyl group with 2 to 20 carbon atoms, an alkynyl group with 2 to 20 carbon atoms, an aryl group with 5 to 20 carbon atoms, an aralkyl group with 6 to 20 carbon atoms, a hydroxyalkyl group with 1 to 20 carbon atoms, a carboxyalkyl group with 1 to 20 carbon atoms, a hydroxyalkyl group with 1 to 20 carbon atoms, an aliphatic carbonyl group with 2 to 20 carbon atoms, an aromatic carbonyl group with 2 to 20 carbon atoms, or the like.)

Example of the sulfur-functionalized benzophenone obtained by the polyether ether ketone decomposition method according to this embodiment includes, but are not limited to, compounds represented by the following formulas.

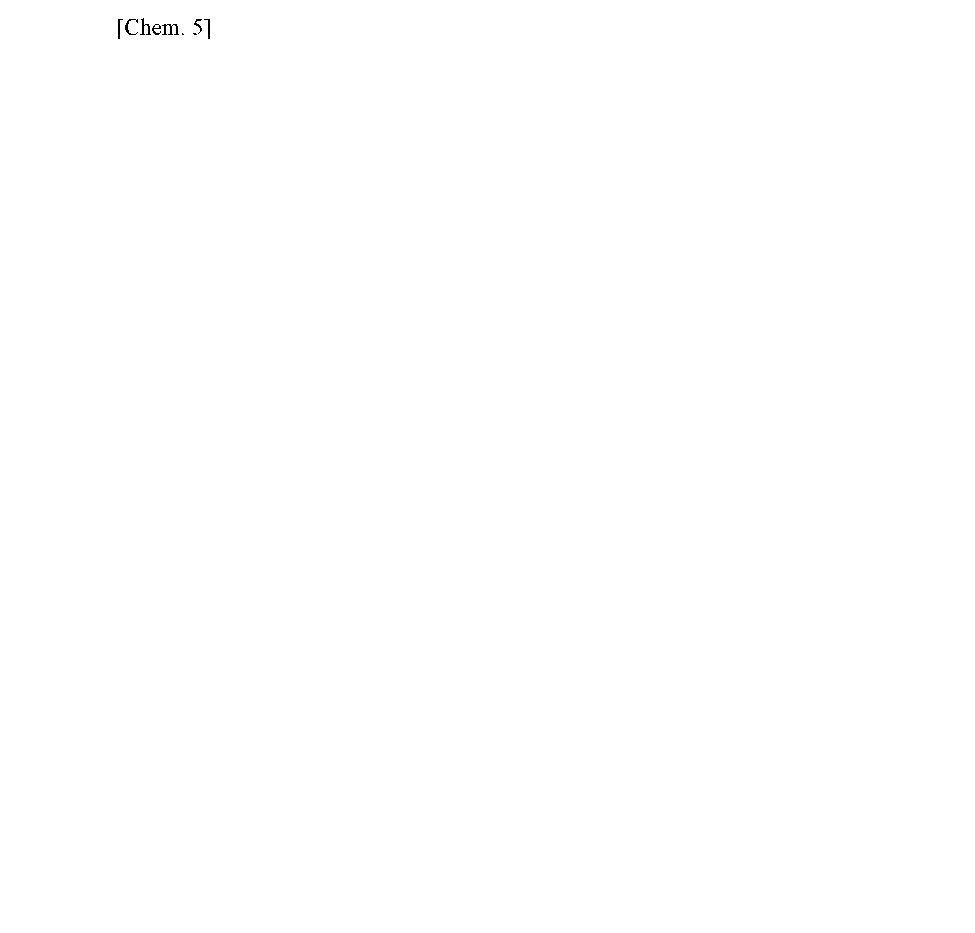

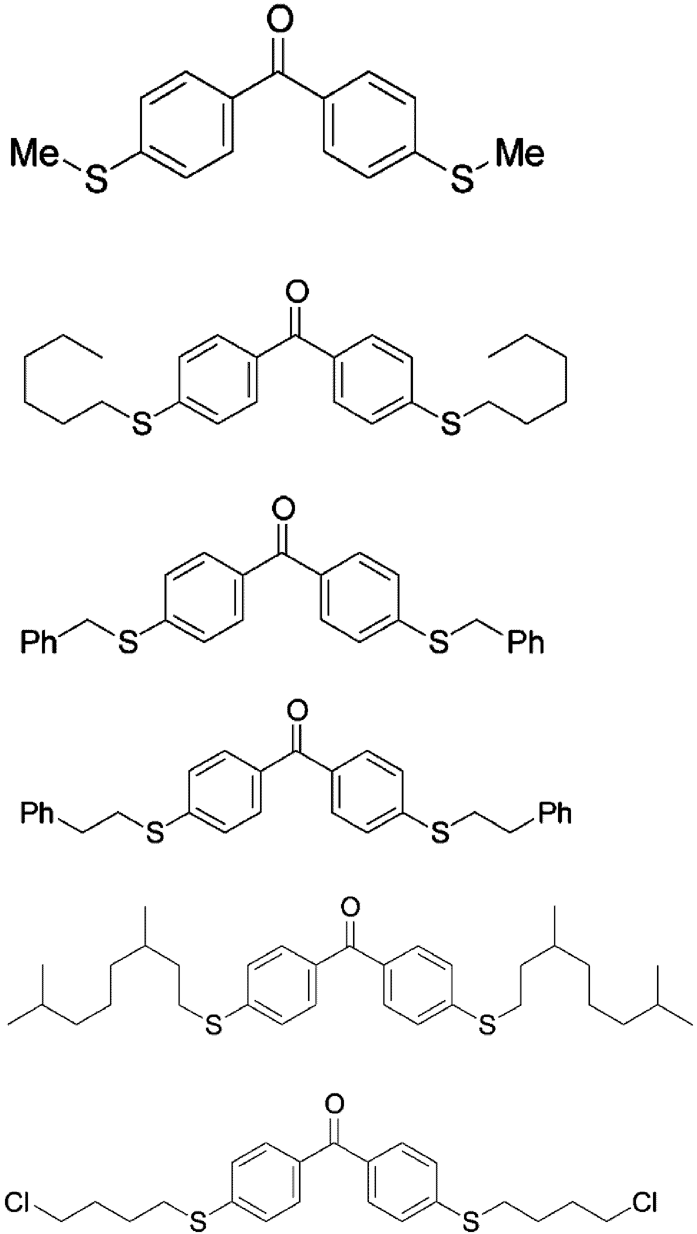

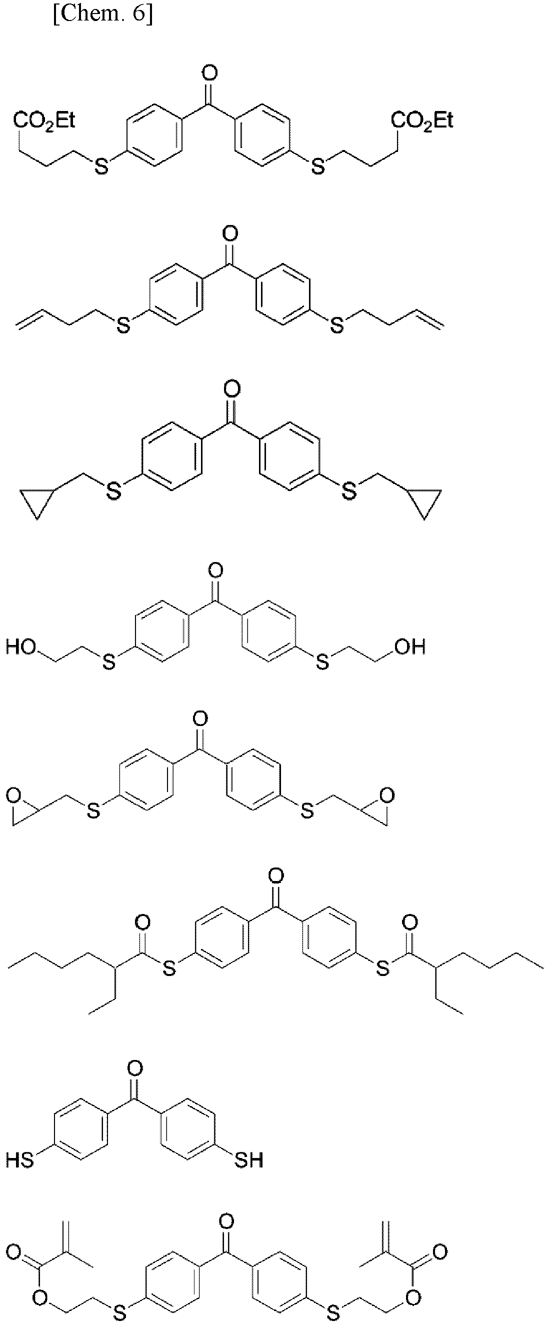

Note that these sulfur-functionalized benzophenones can be produced by: using methyl bromide, methyl iodide, 1-chlorohexane, 1-bromohexane, 1-iodohexane, benzyl chloride, benzyl bromide, benzyl iodide, 2-phenylethyl chloride, 2-phenylethyl bromide, 2-phenylethyl iodide, 1-chloro-3,7-dimethyloctane, 1-bromo-3,7-dimethyloctane, 1-iodo-3,7-dimethyloctane, 1,4-dichlorobenzene, ethyl 4-chlorobutyrate, ethyl 4-bromobutyrate, ethyl 4-iodobutyrate, 4-chloro-1-butene, 4-bromo-1-butene, 4-iodo-1-butene, chloromethylcyclopropane, bromomethylcyclopropane, iodomethylcyclopropane, 2-chloroethanol, 2-bromoethanol, 2-iodoethanol, 3-chloro-1-propene oxide, or 3-bromo-1-propene oxide as the alkyl halide; using 2-ethylhexanoyl chloride or methacryloyl chloride as the acid halide; or also using hydrogen chloride or hydrochloric acid.

The reactions in the first reaction step (a) and second reaction step (b) can also be carried out in the coexistence of glass fibers or various polymers. Examples of the polymers include polyethylene, polypropylene, polystyrene, polyamide, and the like. In addition, composite forms reinforced with fibers, such as carbon-fiber-reinforced polyether ether ketone and glass-fiber-reinforced polyether ether ketone, can also be decomposed by the polyether ether ketone decomposition method according to this embodiment.

According to the polyether ether ketone decomposition method according to this embodiment, hydroquinone can be produced in addition to the sulfur-functionalized benzophenone. In other words, the polyether ether ketone decomposition method according to this embodiment also serves as a production (synthesis) method of a sulfur-functionalized benzophenone and hydroquinone.

The polyether ether ketone decomposition method according to this embodiment will now be specifically and in detail described with reference to examples. Note that the method is not limited to the following examples.

### Examples

### Example 1

In an argon atmosphere, a base (used in the equivalent amount relative to polyether ether ketone shown in Table 1), 1,3-dimethyl-2-imidazolidinone (DMI, 0.2 mL), a thiol (used in the equivalent amount relative to polyether ether ketone shown in Table 1), and a base (used in the equivalent amount relative to polyether ether ketone shown in Table 1) were successively added to 28.8 mg (0.10 mmol, calculated based on the monomer molar weight, average *M*_{w} ~20,800, average *M*ₙ ~10,300, Cat. No. 456640, purchased from Sigma-Aldrich) of powdered polyether ether ketone, and then the reaction mixture was stirred at 150°C.

After 17 to 25 hours, the reaction mixture (first reaction product) was brought to room temperature (25°C), methyl iodide (used in the equivalent amount relative to polyether ether ketone shown in Table 1) was added thereto, and the mixture was stirred at 100°C for 1 hour.

Then, hydrochloric acid (2 M, 1.0 mL) and ethyl acetate were added to the obtained reaction mixture, and an organic layer of the reaction mixture was washed with water and a saturated aqueous sodium chloride solution. The organic layer after extraction was dried by using anhydrous magnesium sulfate and distilled under a reduced pressure to obtain a crude product. The crude product was measured by gas chromatography with adding 3.7 mg (5.0 µL, 0.024 mmol) of undecane and by ¹H NMR with adding 5.2 mg (5.0 µL, 0.059 mmol) of 1,4-dioxane to measure yields of a comonomer A and the target 4,4'-dimethylthiobenzophenone B.

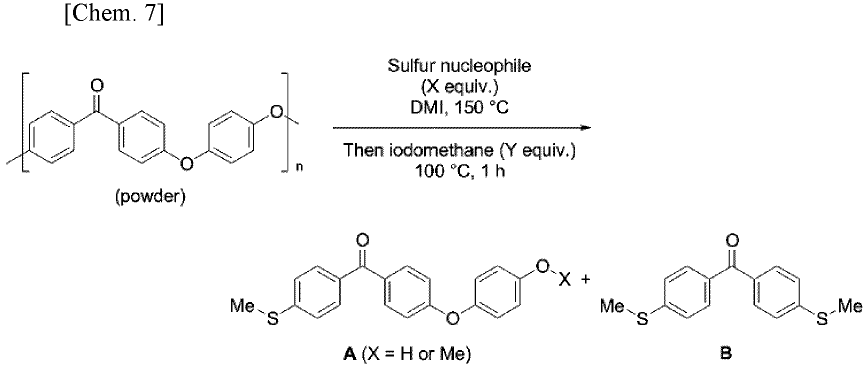

**[Table 1]**

| Entry | Sulfur Reactant / Base (X) | Mel (Y) | time (h) | A (%)* | B (%) |
|---|---|---|---|---|---|
| 1 | PhCH₂CH₂-SH (4) / NaO*t*-Bu (4) | 4 | 25 | 6 | 88 |
| 2 | PhCH₂CH₂-SH (4) / NaO*t*-Bu (4) | 4 | 25 | 9 | 86 |
| 3** | *n*-C₆H₁₃-SH (4) / NaO*t*-Bu (4) | 4 | 17 | trace | 51 |
| 4 | PhCH₂-SH (4) / NaO*t*-Bu (4) | 4 | 22 | 41 | 58 |
| 5 | (*p*-MeC₆H₄)CH₂ (4) / NaO*t*-Bu (4) | 4 | 25 | 47 | 53 |
| 6 | (*o*-MeCₑH₄)CH₂ (4) / NaO*t*-Bu (4) | 4 | 16 | 35 | 65 |
| 7 | PhCH(Me) (4) / NaO*t*-Bu (4) | 4 | 16 | 52 | 37 |
| 8 | 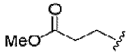 (4) / NaO*t*-Bu (4) | 4 | 15 | 2 | ND*** |
| 9 | 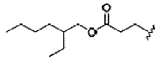 (4)/ NaO*t*-Bu (4) | 4 | 15 | 9 | trace |
| 10 | HSCH₂CH₂ (2) / NaO*t*-Bu (2) | 4 | 22 | 17 | 28 |
| 11 | HOCH₂CH₂ (4) / NaO*t*-Bu (4) | 4 | 18 | 6 | 7 |
| 12 | PhCH₂CH₂-SH (4) / NaO*t*-Bu (3) | 3 | 20 | trace | 93 |
| 13 | PhCH₂CH₂-SH (4) / NaO*t*-Bu (2) | 2 | 24 | 5 | 73 |
| 14 | PhCH₂CH₂-SH (4) / NaOH (3) | 3 | 20 | trace | 95 |
| 15 | PhCH₂CH₂-SH (4) / K₃PO₄ (3) | 3 | 20 | ND*** | 90 |

| | | | | | |
|---|---|---|---|---|---|
| * Total yield of a product in which X=H and a product in which X=Me ** Carried out in an air atmosphere *** Not Detected | | | | | |

As shown in the experimental review in Table 1, depolymerization (Entries 1 to 11) was carried out using various alkanethiols including *n*- hexanethiol, phenylethylthiol, benzylthiol, 2-(methoxycarbonyl)ethylthiol, 1,2-bismercaptoethane, and the like, as well as sodium *tert*-butoxide. As a result, when phenylethylthiol was used, the depolymerization and subsequent decomposition of the comonomer A proceeded efficiently, and 4,4'-dimethylthiobenzophenone B was produced in high yield (Entries 1, 2).

Here, we expected that sodium *tert*-butoxide would exhibit catalytic activity, so we reduced the equivalent amount of the base relative to the thiol and carried out the experiment. Then we found that 4,4'-dimethylthiobenzophenone B could be obtained in high yield by using the base in an amount of 3 equivalents relative to polyether ether ketone (Entry 12). Even when the base was replaced with sodium hydroxide, which is readily available, there was no change in the yield of 4,4'-dimethylthiobenzophenone B (Entry 14).

Note that even when the experiment under the conditions of Entry 2 was carried out in air, the yield of B was not affected (Entry 2). Therefore, it was found that the polyether ether ketone decomposition method according to this embodiment proceeds without problems even in an air atmosphere. Furthermore, hydroquinone was produced in the reaction mixture obtained in all of the tests of Entries 1 to 15.

### Example 2

In this example, depolymerization was carried out under the depolymerization conditions of Entry 12 of Example 1 except that the solvent was replaced with *N,N-*dimethylacetamide.

In an argon atmosphere, 86.5 mg (0.900 mmol) of sodium *tert*-butoxide, 0.6 mL of *N*,*N*-dimethylacetamide (DMAc), and 167 mg (1.21 mmol) of phenylethylthiol were successively added to 86.4 mg (0.30 mmol, calculated based on the monomer molar weight, average *M*_{w} ~20,800, average *M*ₙ ~10,300, Cat. No. 456640, purchased from Sigma-Aldrich) of powdered polyether ether ketone, and then the obtained reaction mixture was stirred at 150°C.

After 20 hours, the reaction mixture was brought to room temperature (25°C), 128 mg (0.900 mmol) of methyl iodide was added thereto, and the mixture was stirred at 100°C for 1 hour. Hydrochloric acid (2 M, 1.0 mL) and 1.5 mL of ethyl acetate were added to the obtained reaction mixture, and an organic layer of the reaction mixture was washed with water and a saturated aqueous sodium chloride solution.

Then, the organic layer after extraction from the reaction mixture was dried by using anhydrous magnesium sulfate and distilled under a reduced pressure to obtain a crude product. From the crude product, 75.9 mg of 4,4'-dimethylthiobenzophenone B was obtained (yield 93%) by silica gel column chromatography (eluent: hexane/ethyl acetate, 96:4 to 7:3). Note that the amount of the comonomer was trace.
¹H NMR (600 MHz, CDCl₃) δ 2.54 (s, 6H, SCH₃), 7.28 (d, *J =* 8.0 Hz, 4H, aromatic), 7.71 (d, *J =* 8.0 Hz, 4H, aromatic). ¹³C NMR (151 MHz, CDCl₃) δ 14.9, 124.8, 130.5, 133.7, 145.0, 195.0. 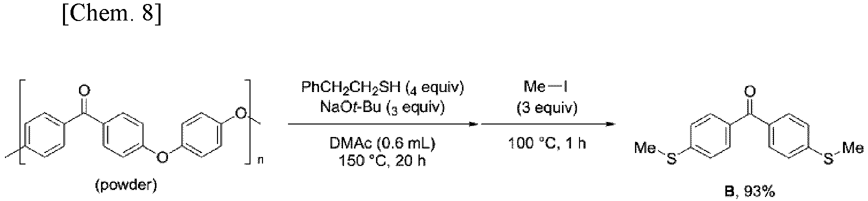 As described above, even when the polyether ether ketone decomposition method according to this embodiment was carried out with replacing the solvent with *N*,*N*-dimethylacetamide, 4,4'-dimethylthiobenzophenone could be obtained in an isolation yield of 93%. In this example as well, hydroquinone was produced in the obtained reaction mixture.

### Example 3

In this example, depolymerization (decomposition reaction) of pelleted polyether ether ketone was carried out.

In an argon atmosphere, 86.5 mg (0.900 mmol) of sodium *tert*-butoxide, 0.6 mL of *N*,*N*-dimethylacetamide (DMAc), and 167 mg (1.21 mmol) of phenylethylthiol were successively added to 86.4 mg (0.30 mmol, calculated based on the monomer molar weight, Cat. No. GF83219856, mean particle size 80 micron, purchased from Sigma-Aldrich) of pelleted polyether ether ketone, and then the reaction mixture was stirred at 150°C.

After 20 hours, the reaction mixture was brought to room temperature (25°C), 128 mg (0.900 mmol) of methyl iodide was added thereto, and the mixture was stirred at 100°C for 1 hour.

Then, the obtained reaction product was measured by ¹H NMR with adding 5.2 mg (5.0 µL, 0.059 mmol) of 1,4-dioxane and dissolving a small amount thereof in deuterated acetone. As a result it was confirmed that polyether ether ketone was decomposed and 4,4'-dimethylthiobenzophenone B and hydroquinone were produced in yields of 94% and 85%, respectively.

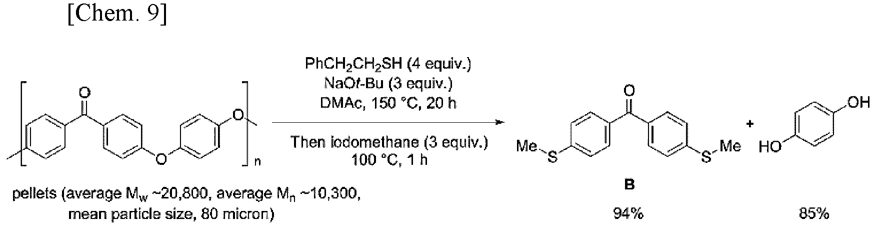

### Example 4

In this example, depolymerization of polyether ether ketone in the form of a film was carried out.

In an argon atmosphere, 86.5 mg (0.900 mmol) of sodium *tert*-butoxide, 0.6 mL of *N*,*N*-dimethylacetamide (DMAc), and 167 mg (1.21 mmol) of phenylethylthiol were successively added to 86.4 mg (0.30 mmol, calculated based on the monomer molar weight, Cat. No. GF55060231-1EA, thickness 0.025 mm, purchased from Sigma-Aldrich) of polyether ether ketone in the form of a light amber transparent film, and then the reaction mixture was stirred at 150°C.

After 20 hours, the reaction mixture was brought to room temperature (25°C), 128 mg (0.900 mmol) of methyl iodide was added thereto, and the mixture was stirred at 100°C for 1 hour.

Then, the obtained reaction product was measured by ¹H NMR with adding 5.2 mg (5.0 µL, 0.059 mmol) of 1,4-dioxane and dissolving a small amount thereof in deuterated acetone. As a result it was confirmed that polyether ether ketone was decomposed and 4,4'-dimethylthiobenzophenone B and hydroquinone were produced in yields of 93% and 85%, respectively.

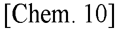

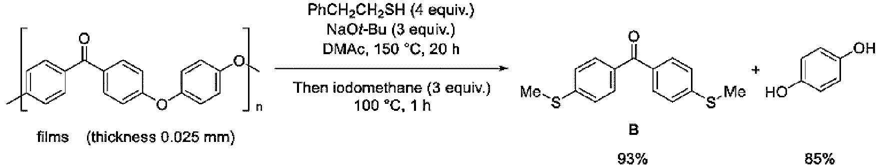

In Examples 3 and 4, depolymerization of pelleted polyether ether ketone and film-like polyether ether ketone was carried out under the depolymerization conditions shown in Example 2. As a result, 4,4'-dimethylthiobenzophenone was obtained in a yield similar to that in the case of powdered polyether ether ketone. In addition, hydroquinone, another depolymerization product, was obtained in a high yield. Since no reaction product of hydroquinone and methyl iodide was observed, it was found that methyl iodide selectively reacted with highly nucleophilic disodium benzophenone-4,4'-dithiolate.

### Example 5

In this example, depolymerization of powdered polyether ether ketone was carried out with added glass fiber.

In an argon atmosphere, 34.2 mg (40 wt%) of glass fiber, 86.4 mg (0.899 mmol) of sodium tert-butoxide, 0.6 mL of *N*,*N*-dimethylacetamide (DMAc), and 167 mg (1.21 mmol) of phenylethylthiol were successively added to 87.2 mg (0.302 mmol, calculated based on the monomer molar weight, average *M*_{w} ~20,800, average *M*ₙ ~10,300, Cat. No. 456640, purchased from Sigma-Aldrich) of powdered polyether ether ketone, and then the reaction mixture was stirred at 150°C.

After 20 hours, the reaction mixture was brought to room temperature (25°C), 128 mg (0.900 mmol) of methyl iodide was added thereto, and the mixture was stirred at 100°C for 1 hour.

Then, the obtained reaction product was measured by ¹H NMR with adding 5.2 mg (5.0 µL, 0.059 mmol) of 1,4-dioxane and dissolving a small amount thereof in deuterated acetone. As a result it was observed that polyether ether ketone was decomposed and 4,4'-dimethylthiobenzophenone B and hydroquinone were produced in yields of 94% and 92%, respectively.

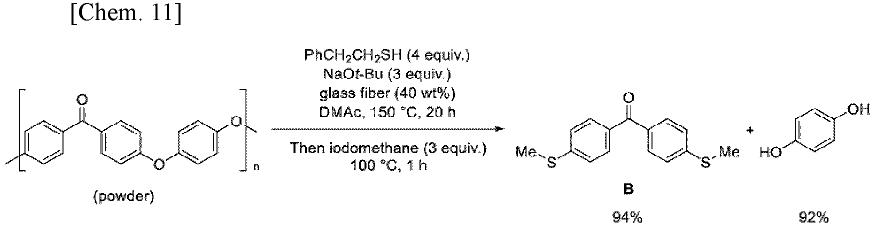

In Example 5, depolymerization was carried out under the depolymerization conditions shown in Example 2 with added glass fiber. As a result, 4,4'-dimethylthiobenzophenone and hydroquinone were obtained in high yields similar to that in the case of pelleted and film-like polyether ether ketone. Similarly, it was confirmed that, when polypropylene (pellet form, isotactic, *M*_{w} ~250,000, Cat. No. 182389, purchased from Sigma-Aldrich), polystyrene (pellet form, polymerization degree about 2,000, Cat. No. 198-12805, purchased from FUJIFILM Wako Pure Chemical Corporation), or nylon 6 (pellet form, Cat. No. 181110, purchased from Sigma-Aldrich) was added in place of glass fiber in depolymerization of pelleted polyether ether ketone, 4,4'-dimethylthiobenzophenone B and hydroquinone were produced in high yields without any problems.

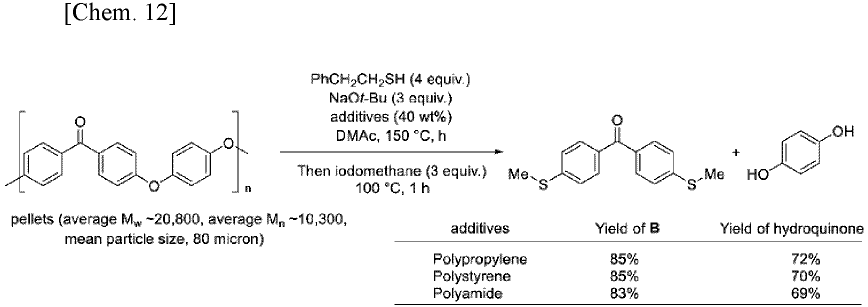

### Example 6

In this example, depolymerization of powdered carbon-fiber-containing polyether ether ketone was carried out.

In an argon atmosphere, 29.0 mg (0.30 mmol) of sodium *tert*-butoxide, 0.2 mL of *N*,*N*-dimethylacetamide (DMAc), and 55.2 mg (0.40 mmol) of phenylethylthiol were successively added to 41.8 mg (PEEK 0.10 mmol, calculated based on the monomer molar weight, TECAPEEK CF30, purchased from Monotaro) of powdered polyether ether ketone containing 30 wt% of carbon fiber, and then the reaction mixture was stirred at 150°C.

After 20 hours, the reaction mixture was brought to room temperature (25°C), 43.3 mg (0.30 mmol) of methyl iodide was added thereto, and the mixture was stirred at 100°C for 1 hour.

Then, hydrochloric acid (2 M, 0.5 mL) and 1.0 mL of ethyl acetate were added to the obtained reaction mixture, and an organic layer of the reaction mixture was washed with water and a saturated aqueous sodium chloride solution.

The organic layer after extraction was dried by using anhydrous magnesium sulfate and distilled under a reduced pressure to obtain a crude product. It was confirmed that, from the crude product, polyether ether keton was decomposed and 18.0 mg of 4,4'-dimethylthiobenzophenone was obtained (yield 65%) by silica gel column chromatography (eluent: hexane/ethyl acetate, 95:5 to 55:45).

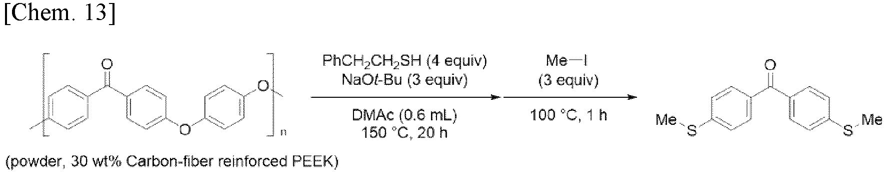

### Example 7

In this example, depolymerization of powdered glass-fiber-containing polyether ether ketone was carried out.

In an argon atmosphere, 28.8 mg (0.30 mmol) of sodium *tert*-butoxide, 0.2 mL of *N*,*N*-dimethylacetamide (DMAc), and 55.2 mg (0.40 mmol) of phenylethylthiol were successively added to 41.0 mg (PEEK 0.10 mmol, calculated based on the monomer molar weight, TECAPEEK CF30, purchased from Monotaro) of powdered polyether ether ketone containing 30 wt% of glass fiber, and then the reaction mixture was stirred at 150°C.

After 20 hours, the reaction mixture was brought to room temperature (25°C), 43.3 mg (0.30 mmol) of methyl iodide was added thereto, and the mixture was stirred at 100°C for 1 hour.

Then, hydrochloric acid (2 M, 0.5 mL) and 1.0 mL of ethyl acetate were added to the obtained reaction mixture, and an organic layer of the reaction mixture was washed with water and a saturated aqueous sodium chloride solution. The organic layer after extraction was dried by using anhydrous magnesium sulfate and distilled under a reduced pressure to obtain a crude product. It was confirmed that, from the crude product, polyether ether keton was decomposed and 14.6 mg of 4,4'-dimethylthiobenzophenone was obtained (yield 53%) by silica gel column chromatography (eluent: hexane/ethyl acetate, 95:5 to 55:45).

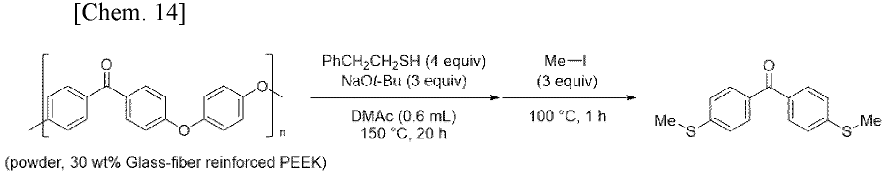

### (Embodiment 2)

The second reaction step was carried out successively after the first reaction step in Embodiment 1, but the present invention is not limited thereto. For example, polyether ether ketone can be decomposed by carrying out only the first reaction step. That is, the first reaction step itself is a polyether ether ketone decomposition method. The polyether ether ketone decomposition method according to this embodiment will now be specifically and in detail described with reference to examples. Note that the method is not limited to the following examples.

### Example 8

In this example, depolymerization was carried out using tert-butylbenzenethiol as a sulfur reactant.

In an argon atmosphere, 1.9 mg (0.020 mmol) of sodium *tert*-butoxide, 0.2 mL of *N*,*N*-dimethylacetamide (DMAc), and 33.7 mg (0.20 mmol) of *tert*-butylbenzenethiol were successively added to 28.9 mg (0.10 mmol, calculated based on the monomer molar weight, average *M*_{w} ~20,800, average *M*ₙ ~10,300, Cat. No. 456640, purchased from Sigma-Aldrich) of powdered polyether ether ketone, and then the reaction mixture was stirred at 150°C.

After 64 hours, the reaction mixture was brought to room temperature (25°C), hydrochloric acid (2 M, 1.0 mL) and 1.5 mL of ethyl acetate were added thereto, and an organic layer of the reaction mixture was washed with water and a saturated aqueous sodium chloride solution. Then, the organic layer after extraction was dried by using anhydrous magnesium sulfate and distilled under a reduced pressure to obtain a crude product. From the crude product, 30.8 mg of 4,4-di(*tert-*butylphenylthio)benzophenone (yield 60%) and 10.8 mg of its comonomer (4-((4-(*tert-*butyl)phenyl)thio)-phenyl) (4-(4-hydroxyphenoxy)phenyl)methanone (yield 24%) by silica gel column chromatography (eluent: hexane/ethyl acetate, 96:4 to 7:3).

NMR measurement result of 4,4-di(tert-butylphenylthio)benzophenone: ¹H NMR (600 MHz, CDCl₃) δ 1.34 (s, 18H, tBu), 7.19 (AA'BB', 4H, aromatic), 7.42-7.46 (m, 8H, aromatic), 7.64 (AA'BB', 4H, aromatic). ¹³C NMR (151 MHz, CDCl₃) δ 31.3, 34.8, 126.7, 126.8, 128.1, 130.6, 134.0, 134.5, 144.9, 152.4, 194.9.

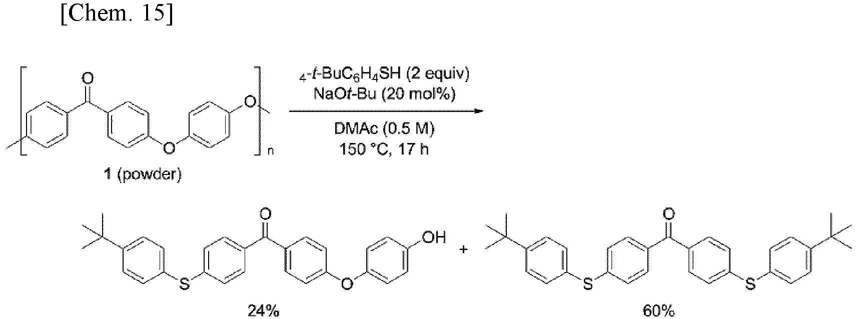

In this example, by using thiols such as *tert*-butylbenzenethiol, in which a carbon functional group on the sulfur is difficult to be eliminated in the reaction system, for example: alkanethiols such as *tert*-butylthiol; thiophenol; and aromatic mercaptans having an electron-donating group such as a *tert*-butyl group, a methyl group, and a methoxy group, a weak electron-withdrawing group such as chlorine and bromine, a benzophenone can be obtained as a first decomposition product having a carbon group derived from thiols.

It was discovered that the base completely acts as a catalyst in this case. In fact, when powdered polyether ether ketone was depolymerized with aromatic mercaptans and a catalytic amount of sodium *tert*-butoxide under the depolymerization conditions shown in Example 8, a novel compound 4,4'-diarylthiobenzophenone and a comonomer which is a precursor thereof were obtained.

Therefore, the polyether ether ketone decomposition method according to the present invention can decompose polyether ether ketone only in the first reaction step.

### Example 9

In an argon atmosphere, 29.7 mg (0.30 mmol) of sodium *tert*-butoxide, 0.2 mL of *N*,*N*-dimethylacetamide (DMAc), 55.6 mg (0.40 mmol) of 2-phenylethylthiol, and 8.7 mg (0.72 mmol) of mesitylene as the internal standard were successively added to 29.3 mg (0.10 mmol, calculated based on the monomer molar weight, average *M*_{w} ~20,800, average *M*ₙ ~10,300, Cat. No. 456640, purchased from Sigma-Aldrich) of powdered polyether ether ketone, and then the reaction mixture was stirred at 100°C.

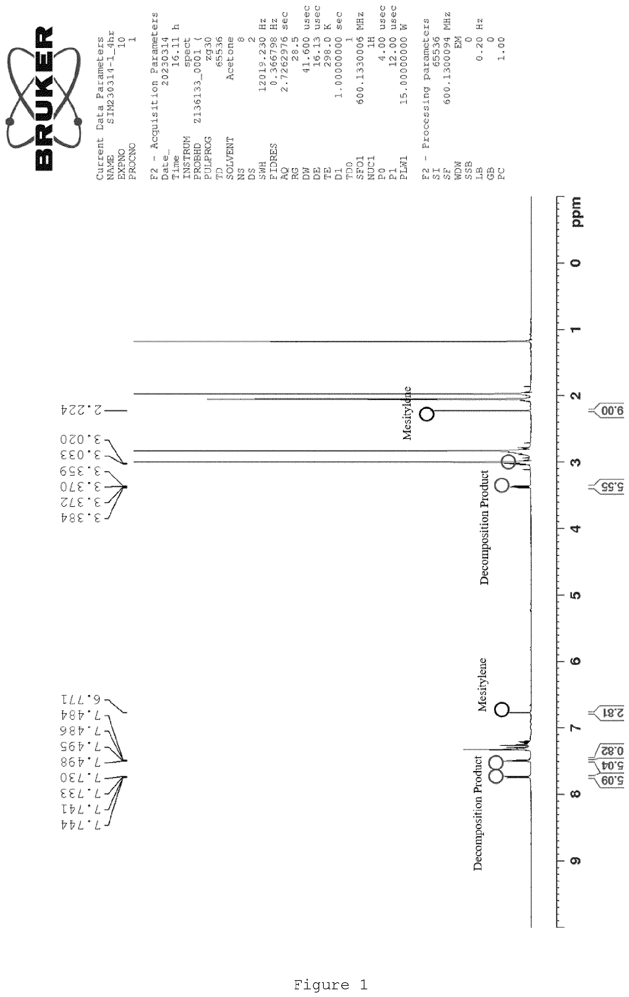

After 4 hours, the reaction mixture was brought to room temperature (25°C), 10 µL of the reaction mixture was taken out, dissolved in 0.5 mL of deuterated acetone, and analyzed by ¹H NMR. As a result, it was confirmed that polyether ether ketone was decomposed and 4,4'-di(2-phenylethylthio)benzophenone was produced as the first decomposition product with a yield of 99% or more. Note that the analysis result of ¹H NMR is shown in Fig. 1.

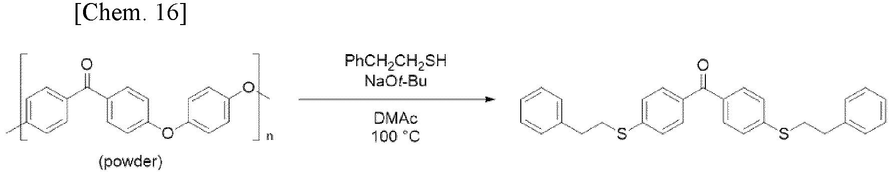

As in this example, even in the case of a thiol having a carbon group that is easily eliminated, such as phenylethylthiol, it is possible to obtain benzophenone, which is the first decomposition product having a carbon group derived from a thiol, by adjusting the reaction conditions.

### Example 10

In this example, it was confirmed that disodium benzophenone-4,4'-dithiolate could be produced as the first decomposition product by the polyether ether ketone decomposition method according to this embodiment.

In an argon atmosphere, 29.7 mg (0.30 mmol) of sodium *tert*-butoxide, 0.2 mL of *N*,*N*-dimethylacetamide (DMAc), and 55.6 mg (0.40 mmol) of 2-phenylethylthiol were successively added to 29.2 mg (0.10 mmol, calculated based on the monomer molar weight, average *M*_{w} ~20,800, average *M*ₙ ~10,300, Cat. No. 456640, purchased from Sigma-Aldrich) of powdered polyether ether ketone, and then the reaction mixture was stirred at 150°C.

After 18 hours, the reaction mixture was brought to room temperature (25°C), and 0.5 mL of ethyl acetate and 0.5 mL of heavy water were added thereto in order to separate the reaction mixture into an organic layer and an aqueous layer.

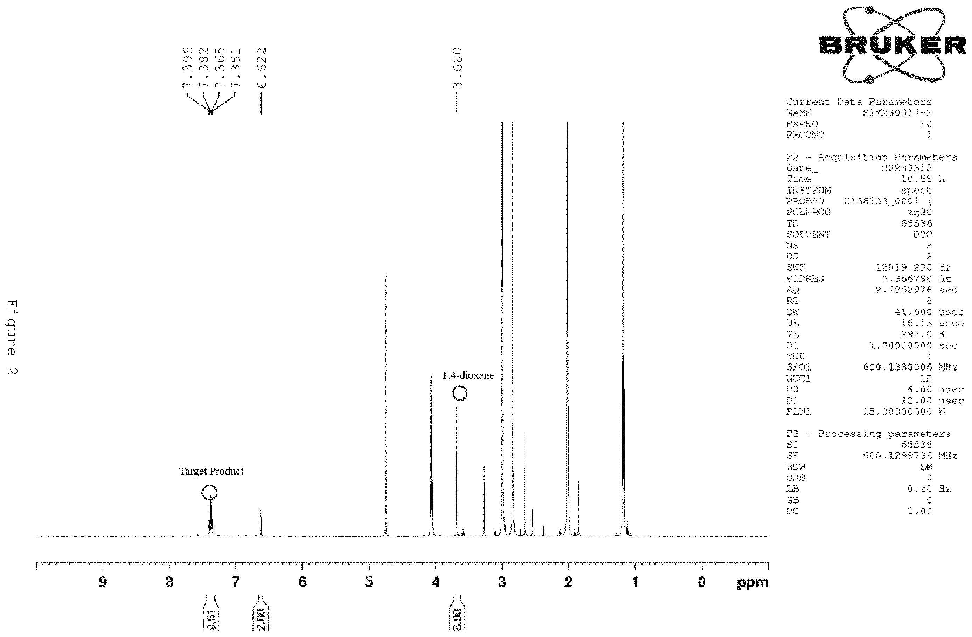

Then, only the aqueous layer was taken out, 5.0 µL (0.059 mmol) of 1,4-dioxane was added thereto as the internal standard, and analysis was carried out by ¹H NMR, confirming that disodium benzophenone-4,4'-dithiolate, which was the first decomposition product, was obtained. Note that the analysis result of ¹H NMR is shown in Fig. 2.

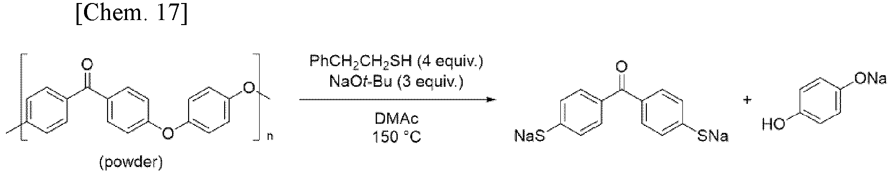

### (Embodiment 3)

In the above-described embodiments, alkanethiols or aromatic mercaptans were used as the sulfur reactant, but the present invention is not limited thereto. Polyether ether ketone can also be decomposed by using sodium sulfide as the sulfur reactant. The polyether ether ketone decomposition method according to this embodiment will now be specifically and in detail described with reference to examples. Note that the method is not limited to the following examples.

### Example 11

In an argon atmosphere, 15.7 mg (0.20 mmol) of sodium sulfide and 0.2 mL of 1,3-dimethyl-2-imidazolidinone (DMI) were successively added to 28.9 mg (0.10 mmol, calculated based on the monomer molar weight, average *M*_{w} ~20,800, average *M*ₙ ~10,300, Cat. No. 456640, purchased from Sigma-Aldrich) of powdered polyether ether ketone, and then the reaction mixture was stirred at 150°C.

After 17 hours, the reaction mixture was brought to room temperature (25°C) to obtain a first decomposition product. Then 59.3 mg (0.42 mmol) of methyl iodide was added to this first decomposition product, and the mixture was stirred at 100°C for 1 hour. Hydrochloric acid (2 M, 0.5 mL) and 1 mL of ethyl acetate were added to the obtained reaction mixture, and an organic layer of the reaction mixture was washed with water and a saturated aqueous sodium chloride solution.

Then, the organic layer after extraction was dried by using anhydrous magnesium sulfate and distilled under a reduced pressure to obtain a crude product.

5.0 µL (0.059 mmol) of 1,4-dioxane as the internal standard, was added to the crude product, and analysis was carried out by ¹H NMR. As a result, it was confirmed that polyether ether ketone was decomposed and 4,4'-dimethylthiobenzophenone was obtained with a yield of 10%.

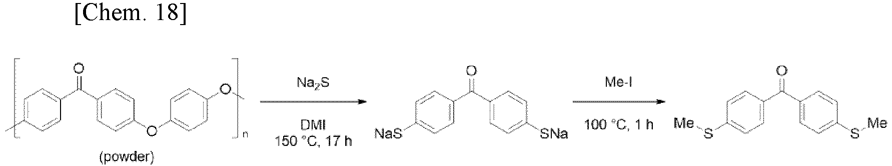

### (Embodiment 4)

The present invention can also decompose polyether ether ketone by using elemental sulfur (S₈) as a sulfur reactant. Note that when elemental sulfur is used as a sulfur reactant, it is necessary to use a strong inorganic base such as NaOH or sodium tert-butoxide in combination. The polyether ether ketone decomposition method according to this embodiment will now be specifically and in detail described with reference to examples. Note that the method is not limited to the following examples.

### Example 12

In an argon atmosphere, 19.4 mg (0.20 mmol, calculated based on the molar weight per sulfur atom) of elemental sulfur, 115 mg (1.2 mmol) of sodium tert-butoxide, and 0.6 mL of 1,3-dimethyl-2-imidazolidinone (DMI) were successively added to 86.4 mg (0.30 mmol, calculated based on the monomer molar weight, average *M*_{w} ~20,800, average *M*ₙ ~10,300, Cat. No. 456640, purchased from Sigma-Aldrich) of powdered polyether ether ketone, and then the reaction mixture was stirred at 150°C.

After 17 hours, the reaction mixture was brought to room temperature (25°C) to obtain a first decomposition product. Then 171 mg (1.2 mmol) of methyl iodide was added to this first decomposition product, and the mixture was stirred at 100°C for 1 hour. Hydrochloric acid (2 M, 1 mL), 2 mL of ethyl acetate, and 5.0 µL (0.024 mmol) of undecane as the internal standard were added to the obtained reaction mixture to separate this reaction mixture into an organic layer and an aqueous layer.

Then, the organic layer after extraction was analyzed by gas chromatography. As a result, it was confirmed that polyether ether ketone was decomposed and 4,4'-dimethylthiobenzophenone was obtained with a yield of 1%.

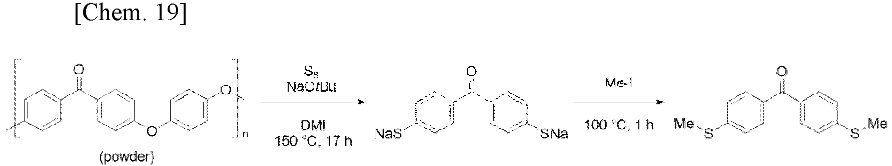

### (Embodiment 5)

In the method of producing (synthesizing) sulfur-functionalized benzophenones from polyether ether ketone in the polyether ether ketone decomposition method according to Embodiment 1, various organic halides other than methyl iodide can be used.

Examples of alkyl halides (R-X) include 1-bromohexane, benzyl bromide, 2-phenylethyl bromide, 2-n-decyl-n-tetradecyl bromide, 1-bromo-3,7-dimethyloctane, 1,4-dichlorobenzene, 4-bromo - 1-butene, 3-bromobutanoic acid ethyl ester, bromomethylcyclopropane, 2-bromoethanol, 3-bromo-1-propene oxide, α-ethylhexanoic acid chloride, hydrogen chloride, and the like. In fact, the following novel compounds can be synthesized from the first decomposition product obtained by the polyether ether ketone decomposition method by using 2-phenylethyl bromide, 2-n-decyl-n-tetradecyl bromide, 1-bromo-3,7-dimethyloctane, 1,4-dichlorobenzene, 4-bromo-1-butene, 3-bromobutanoic acid ethyl ester, bromomethylcyclopropane, 3-bromo-1-propene oxide, or α-ethylhexanoic acid chloride.

### Example 13: Synthesis of 4,4'-di(2-phenylethylthio)benzophenone

In an argon atmosphere, 86.7 mg (0.90 mmol) of sodium *tert*-butoxide, 0.6 mL of *N*,*N*-dimethylacetamide (DMAc), and 167 mg (1.2 mmol) of phenylethylthiol were successively added to 86.4 mg (0.30 mmol, calculated based on the monomer molar weight, average *M*_{w} ~20,800, average *M*ₙ ~10,300, Cat. No. 456640, purchased from Sigma-Aldrich) of powdered polyether ether ketone, and then the reaction mixture was stirred at 150°C.

After 20 hours, the reaction mixture (first decomposition product) was brought to room temperature (25°C), 167 mg (0.90 mmol) of phenylethyl bromide was added thereto, and the mixture was stirred at room temperature (25°C) for 4 hours.

Then, 1.5 mL of water and 1.5 mL of ethyl acetate were added to the obtained reaction mixture, and an organic layer of the reaction mixture was washed with water and a saturated aqueous sodium chloride solution. The organic layer after extraction was dried by using anhydrous magnesium sulfate and distilled under a reduced pressure to obtain a crude product. From the crude product, 106 mg of the target 4,4'-di(2-phenylethylthio)benzophenone was obtained (yield 78%) by silica gel column chromatography (eluent: hexane/ethyl acetate, 96:4 to 7:3).
¹H NMR (600 MHz, CDCl₃) δ 3.00 (t, *J=* 7.5 Hz, 4H, CH₂), 3.27 (t, *J=* 8.1 Hz, 4H, CH₂), 7.23-7.27 (m, 6H, aromatic), 7.33 (AA'BB'C, 4H, aromatic), 7.36 (AA'BB', 4H, aromatic), 7.72 (AA'BB', 4H, aromatic). ¹³C NMR (151 MHz, CDCl₃) δ 33.6, 35.2, 126.5, 126.7, 128.6, 128.7, 130.6, 134.3, 139.8, 143.4, 195.0.

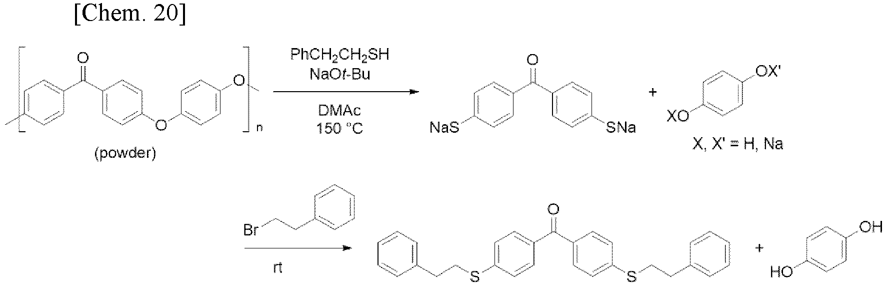

### Example 14: Synthesis of 4,4'-di(2-norm-decyl-norm-tetradecylthio)benzophenone

In an argon atmosphere, 86.7 mg (0.90 mmol) of sodium *tert*-butoxide, 0.6 mL of *N*,*N*-dimethylacetamide (DMAc), and 167 mg (1.2 mmol) of phenylethylthiol were successively added to 86.9 mg (0.30 mmol, calculated based on the monomer molar weight, average *M*_{w} ~20,800, average *M*ₙ ~10,300, Cat. No. 456640, purchased from Sigma-Aldrich) of powdered polyether ether ketone, and then the reaction mixture was stirred at 150°C.

After 20 hours, the reaction mixture (first decomposition product) was brought to room temperature (25°C), 376 mg (0.90 mmol) of 2-n-decyl-n-tetradecyl bromide was added thereto, and the mixture was stirred at 80°C for 2 hours.

Then, 1.5 mL of water, 1.5 mL of hexane, and 1.5 mL of ethylene chloride were added to the obtained reaction mixture, and an organic layer of the reaction mixture was washed with water and a saturated aqueous sodium chloride solution. The organic layer after extraction was dried by using anhydrous magnesium sulfate and distilled under a reduced pressure to obtain a crude product. From the crude product, 240 mg of the target 4,4'-di(2-norm-decyl-norm-tetradecylthio)benzophenone was obtained (yield 87%) by silica gel column chromatography (eluent: hexane/ethyl acetate, 100:0 to 7:3) and preparative liquid chromatography (eluent: chloroform).
¹H NMR (600 MHz, CDCl₃) δ 0.87 (t, *J=* 6.9 Hz, 12H, methyl), 1.22-1.33 (m, 72H, methylene), 1.36-1.45 (m, 8H, methylene), 1.66-1.71 (m, 2H, methylene), 2.97 (d, *J =* 6.4 Hz, 4H, SCH₂), 7.32 (AA'BB', 4H, aromatic), 7.69 (AA'BB', 4H, aromatic). ¹³C NMR (151 MHz, CDCl₃) δ 14.1, 22.7, 26.6, 29.36, 29.38, 29.62, 29.65, 29.68, 29.69, 29.7, 29.9, 31.93, 31.94, 33.3, 37.0, 37.4, 126.3, 130.4, 134.1, 144.7, 194.9 (Several signals derived from alkyl carbons are overlapping with other signals.).

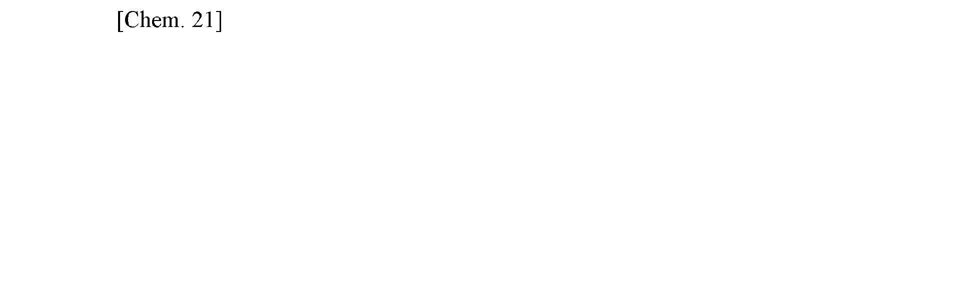

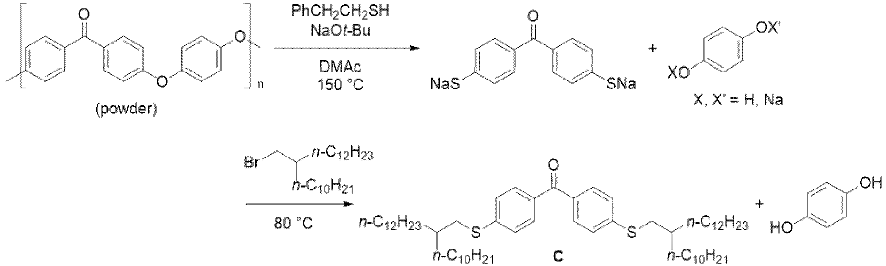

### Example 15: Synthesis of 4,4'-di(4-chlorobutylthio)benzophenone

In an argon atmosphere, 86.9 mg (0.90 mmol) of sodium *tert*-butoxide, 0.6 mL of *N*,*N*-dimethylacetamide (DMAc), and 167 mg (1.2 mmol) of phenylethylthiol were successively added to 86.4 mg (0.30 mmol, calculated based on the monomer molar weight, average *M*_{w} ~20,800, average *M*ₙ ~10,300, Cat. No. 456640, purchased from Sigma-Aldrich) of powdered polyether ether ketone, and then the reaction mixture was stirred at 150°C.

After 20 hours, the reaction mixture (first decomposition product) was brought to room temperature (25°C), 383 mg (3.0 mmol) of 1,4-dichlorobutane was added thereto, and the mixture was stirred at 80°C for 23 hours.

Then, water and ethylene chloride were added to the obtained reaction mixture, and an organic layer of the reaction mixture was washed with water and a saturated aqueous sodium chloride solution. The organic layer after extraction was dried by using anhydrous magnesium sulfate and distilled under a reduced pressure to obtain a crude product. From the crude product, 85.6 mg of the target 4,4'-di(4-chlorobutylthio)benzophenone was obtained (yield 67%) by preparative liquid chromatography (eluent: chloroform).
¹H NMR (600 MHz, CDCl₃) δ 1.86-1.91 (m, 4H, methylene), 1.93-1.98 (m, 4H, methylene), 3.04 (t, *J =* 7.1 Hz, 4H, SCH₂), 3.57 (d, *J =* 6.4 Hz, 4H, ClC*H*₂), 7.33 (AA'BB', 4H, aromatic), 7.71 (AA'BB', 4H, aromatic). ¹³C NMR (151 MHz, CDCl₃) δ 26.1, 31.4, 31.5, 44.3, 126.6, 130.5, 134.4, 143.3, 194.8.

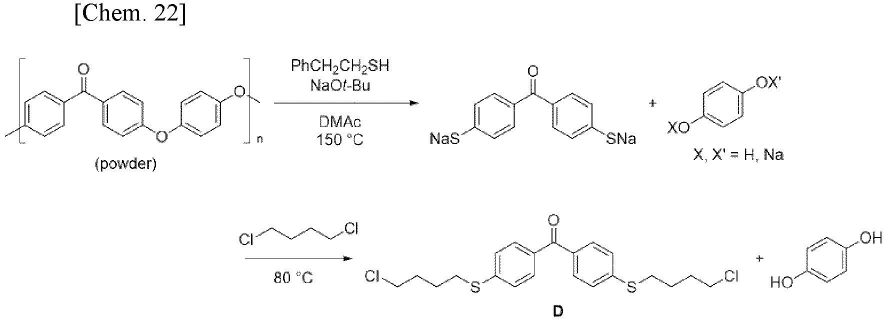

### Example 16: Synthesis of 4,4'-di(4-butenylthio)benzophenone

In an argon atmosphere, 86.5 mg (0.90 mmol) of sodium *tert*-butoxide, 0.6 mL of *N*,*N*-dimethylacetamide (DMAc), and 167 mg (1.2 mmol) of phenylethylthiol were successively added to 86.5 mg (0.30 mmol, calculated based on the monomer molar weight, average *M*_{w} ~20,800, average *M*ₙ ~10,300, Cat. No. 456640, purchased from Sigma-Aldrich) of powdered polyether ether ketone, and then the reaction mixture was stirred at 150°C.

After 20 hours, the reaction mixture (first decomposition product) was brought to room temperature (25°C), 122 mg (0.9 mmol) of 4-bromo-1-butene was added thereto, and the mixture was stirred at 100°C for 2 hours.

Then, water and ethyl acetate were added to the obtained reaction mixture, and an organic layer of the reaction mixture was washed with water and a saturated aqueous sodium chloride solution. The organic layer after extraction was dried by using anhydrous magnesium sulfate and distilled under a reduced pressure to obtain a crude product. From the crude product, 102 mg of the target 4,4'-di(4-butenylthio)benzophenone was obtained (yield 97%) by silica gel column chromatography (eluent: hexane/ethyl acetate, 100:0 to 7:3).
¹H NMR (600 MHz, CDCl₃) δ 2.44-2.48 (m, 4H, CH₂), 3.07 (t, *J =* 7.6 Hz, 4H, SCH₂), 5.09 (dq, *J=* 1.4, 10.1 Hz, 2H, ethenyl), 5.13 (dq, *J=* 1.6, 17.1 Hz, 2H, ethenyl), 5.13 (ddt, *J=* 6.7, 10.4, 17.1 Hz, 2H, ethenyl), 7.33 (AA'BB'C, 4H, aromatic), 7.71 (AA'BB', 4H, aromatic). ¹³C NMR (151 MHz, CDCl₃) δ 31.4, 33.0, 116.8, 126.4, 130.6, 134.3, 135.9, 143.5, 195.0.

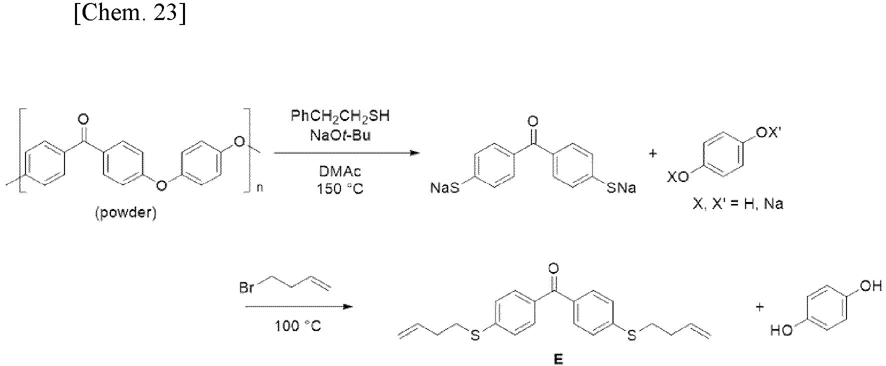

### Example 17: Synthesis of 4,4'-di(3-ethoxycarbonylpropylthio)benzophenone

In an argon atmosphere, 86.5 mg (0.90 mmol) of sodium *tert*-butoxide, 0.6 mL of *N*,*N*-dimethylacetamide (DMAc), and 167 mg (1.2 mmol) of phenylethylthiol were successively added to 86.4 mg (0.30 mmol, calculated based on the monomer molar weight, average *M*_{w} ~20,800, average *M*ₙ ~10,300, Cat. No. 456640, purchased from Sigma-Aldrich) of powdered polyether ether ketone, and then the reaction mixture was stirred at 150°C.

After 20 hours, the reaction mixture (first decomposition product) was brought to room temperature (25°C), 177 mg (0.9 mmol) of ethyl 4-bromobutyrate was added thereto, and the mixture was stirred at 100°C for 2 hours.

Then, of water and ethyl acetate were added to the obtained reaction mixture, and an organic layer of the reaction mixture was washed with water and a saturated aqueous sodium chloride solution. The organic layer after extraction was dried by using anhydrous magnesium sulfate and distilled under a reduced pressure to obtain a crude product. From the crude product, 127 mg of the target 4,4'-di(3-ethoxycarbonylpropylthio)benzophenone was obtained (yield 89%) by silica gel column chromatography (eluent: hexane/ethyl acetate, 96:4 to 7:3).
¹H NMR (600 MHz, CDCl₃) δ 1.26 (t, *J =* 7.2 Hz, 6H, CH₃), 2.03 (quint, *J =* 7.2 Hz, 4H, CH₂), 2.49 (t, *J=* 7.4 Hz, 4H, CH₂), 3.06 (t, *J=* 7.0 Hz, 4H, SCH₂), 4.14 (q, *J = 7.2* Hz, 4H, OCH₂), 7.35 (AA'BB', 4H, aromatic), 7.70 (AA'BB', 4H, aromatic). ¹³C NMR (151 MHz, CDCl₃) δ 14.3, 24.1, 31.4, 32.9, 60.6, 126.6, 130.6, 134.4, 143.1, 172.8, 194.9.

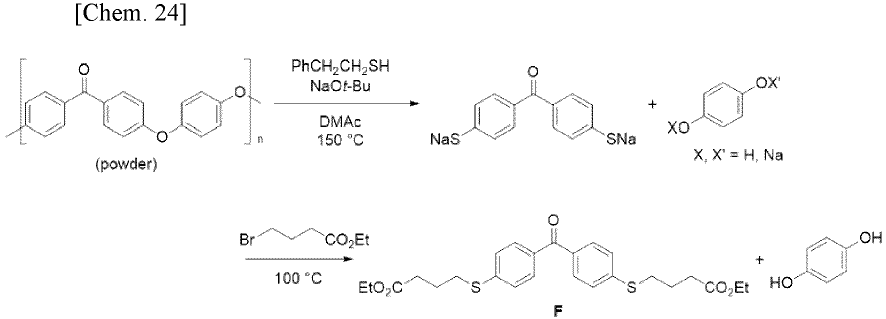

### Example 18: Synthesis of 4,4'-di(cyclopropylmethylthio)benzophenone

In an argon atmosphere, 86.5 mg (0.90 mmol) of sodium *tert*-butoxide, 0.6 mL of *N*,*N*-dimethylacetamide (DMAc), and 167 mg (1.2 mmol) of phenylethylthiol were successively added to 86.4 mg (0.30 mmol, calculated based on the monomer molar weight, average *M*_{w} ~20,800, average *M*ₙ ~10,300, Cat. No. 456640, purchased from Sigma-Aldrich) of powdered polyether ether ketone, and then the reaction mixture was stirred at 150°C.

After 20 hours, the reaction mixture (first decomposition product) was brought to room temperature (25°C), 122 mg (0.9 mmol) of bromomethylcyclopropane was added thereto, and the mixture was stirred at room temperature (25°C) for 3.5 hours.

Then, water and ethyl acetate were added to the obtained reaction mixture, and an organic layer of the reaction mixture was washed with water and a saturated aqueous sodium chloride solution. The organic layer after extraction was dried by using anhydrous magnesium sulfate and distilled under a reduced pressure to obtain a crude product. From the crude product, 98 mg of the target 4,4'-di(cyclopropylmethylthio)benzophenone was obtained (yield 92%) by silica gel column chromatography (eluent: hexane/ethyl acetate, 96:4 to 7:3).
¹H NMR (600 MHz, CDCl₃) δ 0.30-0.33 (m, 4H, cCH₂), 0.62-0.65 (m, 4H, cCH₂), 1.08-1.14 (m, 2H, cCH), 2.96 (d, *J =* 7.0 Hz, 4H, SCH₂), 7.34 (AA'BB', 4H, aromatic), 7.69 (AA'BB', 4H, aromatic). ¹³C NMR (151 MHz, CDCl₃) δ 5.83, 10.1, 38.0, 126.4, 130.5, 134.2, 144.2, 195.0.

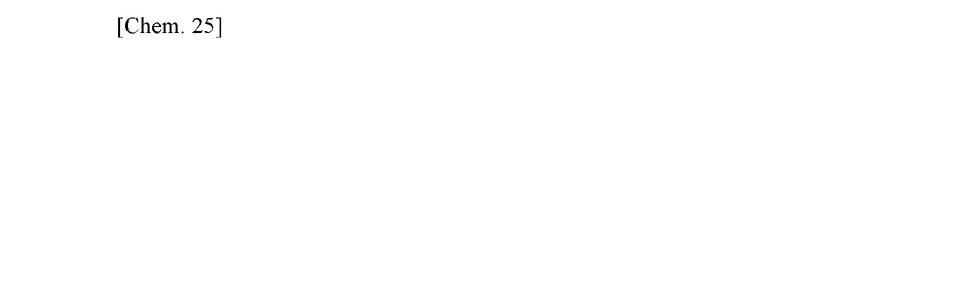

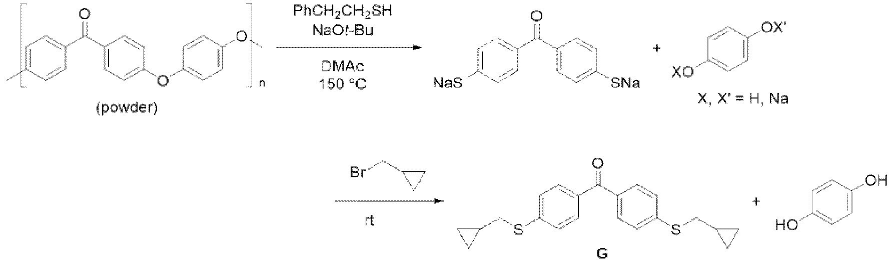

### Example 19: Synthesis of 4,4'-di(4-oxiranylmethylthio)benzophenone

In an argon atmosphere, 87.4 mg (0.91 mmol) of sodium *tert*-butoxide, 0.6 mL of *N*,*N*-dimethylacetamide (DMAc), and 167 mg (1.2 mmol) of phenylethylthiol were successively added to 86.4 mg (0.30 mmol, calculated based on the monomer molar weight, average *M*_{w} ~20,800, average *M*ₙ ~10,300, Cat. No. 456640, purchased from Sigma-Aldrich) of powdered polyether ether ketone, and then the reaction mixture was stirred at 150°C.

After 20 hours, the reaction mixture (first decomposition product) was brought to room temperature (25°C), 411 mg (3.0 mmol) of 3-bromo-1-propene oxide was added thereto while further cooling to 0°C in an ice bath, and the mixture was stirred at room temperature (25°C) for 20 hours.

Then, water and methylene chloride were added to the obtained reaction mixture, and an organic layer of the reaction mixture was washed with water and a saturated aqueous sodium chloride solution. The organic layer after extraction was dried by using anhydrous magnesium sulfate and distilled under a reduced pressure to obtain a crude product. From the crude product, 58 mg of the target 4,4'-di(4-oxiranylmethylthio)benzophenone was obtained (yield 53%) by silica gel column chromatography (eluent: hexane/ethyl acetate, 100:0 to 7:3) and preparative liquid chromatography (eluent: chloroform).
¹H NMR (600 MHz, CDCl₃) 2.65 (dd, *J =* 2.6, 4.8 Hz, 2H, methylene), 2.83-2.85 (m, 2H, methylene), 3.14 (dd, *J=* 6.8, 15.8 Hz, 2H, methylene), 3.22-3.25 (m, 4H), 7.43 (AA'BB', 4H, aromatic), 7.71 (AA'BB', 4H, aromatic). ¹³C NMR (151 MHz, CDCl₃) 35.0, 47.3, 50.7, 127.4, 130.6, 134.9, 142.3, 194.8.

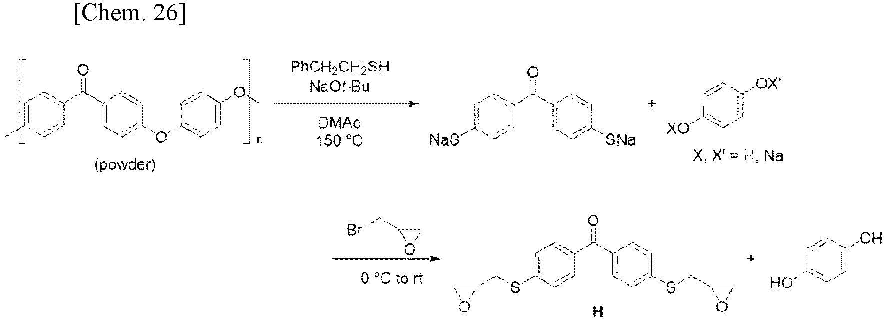

### Example 20: Synthesis of 4,4'-bis(3,7-dimethyloctylthio)benzophenone

In an argon atmosphere, 1.15 g (12.0 mmol) of sodium *tert*-butoxide, 8.0 mL of N,N-dimethylacetamide (DMAc), and 2.21 g (16.0 mmol) of phenylethylthiol were successively added to 1.15 g (3.99 mmol, calculated based on the monomer molar weight, average *M*_{w} ~20,800, average *M*ₙ ~10,300, Cat. No. 456640, purchased from Sigma-Aldrich) of powdered polyether ether ketone, and then the reaction mixture was stirred at 150°C.

After 22 hours, the reaction mixture (first decomposition product) was brought to room temperature (25°C), 2.65 mg (12.0 mmol) of 1-bromo-3,7-dimethyloctane was added thereto, and the mixture was stirred at 100°C for 17 hours.

Then, hydrochloric acid (2 M, 10 mL) and 20 mL of ethyl acetate were added to the obtained reaction mixture, and an organic layer of the reaction mixture was washed with water and a saturated aqueous sodium chloride solution. The organic layer after extraction was dried by using anhydrous magnesium sulfate and distilled under a reduced pressure to obtain a crude product. From the crude product, 1.70 g of 4,4'-bis(3,7-dimethyloctylthio)benzophenone was obtained (yield 81%) by silica gel column chromatography (eluent: hexane/ethyl acetate, 96:4 to 7:3) and vacuum drying at 170°C (ca. 0.5 Torr).
¹H NMR (600 MHz, CDCl₃) δ 0.86 (d, *J =* 6.6 Hz, 12H, methyl), 0.94 (d, *J =* 6.6 Hz, 6H, methyl), 1.12-1.16 (m, 6H, methylene), 1.22-1.34 (m, 6H, methylene), 1.50-1.56 (m, 4H, methylene), 1.58-1.63 (m, 2H, methylene), 1.69-1.75 (m, 2H, methylene), 2.97 (ddd, *J=* 6.3, 9.6, 12.5 Hz, 2H, SCH₂), 3.05 (ddd, *J =* 6.3, 9.6, 12.5 Hz, 2H, SCH₂), 7.32 (AA'BB', 4H, aromatic), 7.70 (AA'BB', 4H, aromatic). ¹³C NMR (151 MHz, CDCl₃) δ 19.4, 22.6, 22.7, 24.7, 28.0, 30.0, 32.4, 35.9, 36.9, 39.2, 126.2, 130.5, 134.2, 144.1, 194.9.

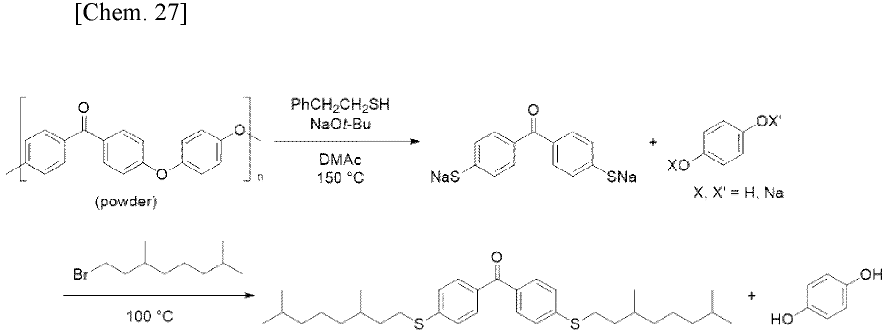

### Example 21: Synthesis of 4,4'-di(1-ethyl-n-pentylcarbonylthio)benzophenone

In an argon atmosphere, 87.0 mg (0.91 mmol) of sodium *tert*-butoxide, 0.6 mL of N,N-dimethylacetamide (DMAc), and 167 mg (1.2 mmol) of phenylethylthiol were successively added to 86.1 mg (0.30 mmol, calculated based on the monomer molar weight, average *M*_{w} ~20,800, average *M*ₙ ~10,300, Cat. No. 456640, purchased from Sigma-Aldrich) of powdered polyether ether ketone, and then the reaction mixture was stirred at 150°C.

After 22 hours, the reaction mixture (first decomposition product) was brought to room temperature (25°C), 147 mg (0.91 mmol) of 2-ethylhexanoyl chloride and 0.6 mL of tetrahydrofuran were added thereto, and the mixture was stirred at room temperature (25°C) for 5 hours.

Then, 5 mL of hydrochloric acid (0.5 M) and 5 mL of ethyl acetate were added to the obtained reaction mixture, and an organic layer of the reaction mixture was washed with water and a saturated aqueous sodium chloride solution. The organic layer after extraction was dried by using anhydrous magnesium sulfate and distilled under a reduced pressure to obtain a crude product. From the crude product, 121 mg of the target 4,4'-di(1-ethyl-n-pentylcarbonylthio)benzophenone was obtained (yield 81%) by silica gel column chromatography (eluent: hexane/ethyl acetate, 100:0 to 7:3) and preparative liquid chromatography (eluent: chloroform).

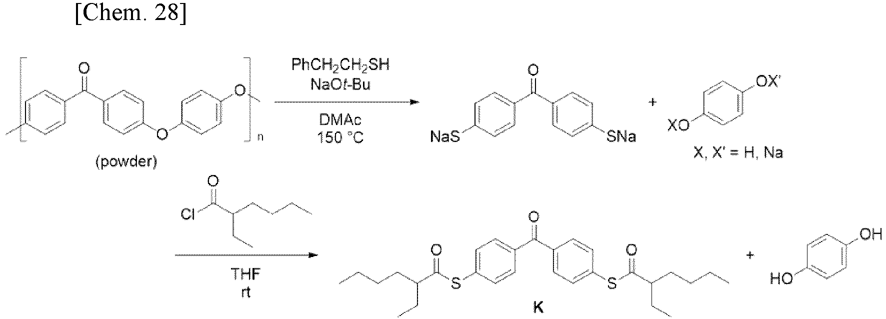

### (Other Embodiments)

The organic solvent in the above-described embodiments does not contain water, but the present invention is not limited thereto. The organic solvent may contain water.

Moreover, in the first reaction step of the above-described embodiments, a base and one of the following substances: alkanethiols, aromatic mercaptans, sodium sulfide, and elemental sulfur are reacted with polyether ether ketone, but the present invention is not limited thereto. In the first reaction step, a base and more than one of the following: alkanethiols, aromatic mercaptans, sodium sulfide, and elemental sulfur may be reacted with polyether ether ketone.

Furthermore, in the second reaction step of the above-described embodiments, one of the following substances: an alkyl halide, an acid halide, and hydrogen chloride is reacted with the first reaction product, but the present invention is not limited thereto. In the second reaction step, more than one of the following: an alkyl halide, an acid halide, and hydrogen chloride may be reacted with the first reaction product.

### Industrial Applicability

The decomposition method of the present invention provides sulfur-functionalized benzophenones and hydroquinone from polyether ether ketone in good yields. Various benzophenones can be obtained depending on the type of organic halides used after depolymerization. Therefore, the depolymerization reaction in the present invention can be utilized as a technology to obtain various useful organic substances from polyether ether ketone.

## Claims

1. A polyether ether ketone decomposition method for decomposing polyether ether ketone, **characterized in that** the method comprises a first reaction step of reacting the polyether ether ketone with a base and at least one of an alkanethiol, an aromatic mercaptan, sodium sulfide, and elemental sulfur in an organic solvent.

2. The polyether ether ketone decomposition method according to claim 1, **characterized in that** the at least one of the alkanethiol, the aromatic mercaptan, sodium sulfide, and elemental sulfur is reacted in an amount of 0.1 to 6 equivalents relative to the polyether ether ketone.

3. The polyether ether ketone decomposition method according to claim 1, **characterized in that** the base is reacted in an amount of 2 to 6 equivalents relative to the polyether ether ketone.

4. The polyether ether ketone decomposition method according to any one of claims 1 to 3, **characterized in that** the first reaction step is carried out at 100 to 200°C.

5. The polyether ether ketone decomposition method according to claim 1, **characterized in that** the base is at least one selected from a group consisting of sodium hydroxide, potassium hydroxide, potassium carbonate, potassium phosphate, cesium carbonate, sodium *tert*-butoxide, lithium *tert*-butoxide, potassium *tert*-butoxide, lithium bis(trimethylsilyl)amide, sodium bis(trimethylsilyl)amide, potassium bis(trimethylsilyl)amide, a phosphazene base, *t*-Bu-P₄ (1-*tert*-butyl-4,4,4-tris(dimethylamino)-2,2-bis[tris(dimethylamino)-phosphoranylideneamino]-2λ⁵,4λ⁵-catenadi(phosphazene)), *t*-Oct-P₄ (1-tert-octyl-4,4,4-tris(dimethylamino)-2,2-bis[tris(dimethylamino)-phosphoranylideneamino]-2λ⁵,4λ⁵-catenadi(phosphazene)), and *t*-Bu-P₂ (1-*tert*-butyl-2,2,4,4,4-pentakis(dimethylamino)-2λ⁵,4λ⁵-catenadi(phosphazene)).

6. The polyether ether ketone decomposition method according to claim 1, **characterized in that** the organic solvent is at least one selected from a group consisting of 1,3-dimethyl-2-imidazolidinone, N,N-dimethylacetamide, N,N-dimethylformamide, N-methyl-2-pyrrolidone, benzonitrile, and 1,4-dioxane.

7. A polyether ether ketone decomposition method, **characterized in that** the method comprises a second reaction step of reacting a first reaction product obtained by the first reaction step according to claim 1 with at least one of an alkyl halide, an acid halide, and hydrogen chloride.

8. The polyether ether ketone decomposition method according to claim 7, **characterized in that**:
the alkyl halide is at least one selected from a group consisting of methyl iodide, 1-bromohexane, benzyl bromide, 2-phenylethyl bromide, 11-bromomethyltricosane, 1-bromo-3,7-dimethyloctane, 1,4-dichlorobenzene, 4-bromo-1-butene, 3-bromobutanoic acid ethyl ester, bromomethylcyclopropane, 2-bromoethanol, and 3-bromo-1-propene oxide; and
the acid halide is at least one selected from a group consisting of acetyl chloride, benzoyl chloride, and α-ethylhexanoic acid chloride.

9. A compound represented by any one of formulas (1) to (11), **characterized in that** the compound is synthesized by using a product obtained by the polyether ether ketone decomposition method according to claim 1.
